# EUROPÄISCHE PATENTSCHRIFT

(11) **EP 0 799 830 B1**
(45) Veröffentlichungstag und Bekanntmachung des Hinweises auf die Patenterteilung: **29.05.2002**
(21) Anmeldenummer: 97810141.8
(22) Anmeldetag: 13.03.1997
(51) Int. Cl.: C07D 513/04, C09K 19/34

(54) **Imidazothiazol-Derivate als komponente flüssigkristalline Mischungen**
Imidazothiazol derivatives as components of liquid crystalline mixtures
Dérivés d'imidazothiazoles comme constituants de mélanges de cristaux liquides

(30) Priorität: 04.04.1996 CH 88796
(43) Veröffentlichungstag der Anmeldung: 08.10.1997
(73) Patentinhaber: Rolic AG, 6301 Zug (CH)
(72) Erfinder: Buchecker, Richard, Dr., 8008 Zürich (CH); Friedrichsen, Willy, Prof. Dr,, 24601 Wankendorf (DE); Fünfschilling, Jürg, Prof. Dr., 4054 Basel (CH); Lüpfert, Sandra, 24103 Kiel (DE)
(74) Vertreter: Liebetanz, Michael, Dipl.-Phys.

(56) Entgegenhaltungen:
- EP-A- 0 144 013
- DE-A- 3 734 332
- ARCH. PHARM. CHEMI, SCI. ED. (AVPCCS,0302248X);87; VOL.15 (2); PP.41-9, UNIV. BOLOGNA;IST. CHIM. FARM. TOSSICOL.; BOLOGNA; I-40126; ITALY (IT), XP000677221 ANDREANI A ET AL: "Synthesis and mutagenic activity of imidazo[2,1-b]thiazoles bearing at least one nitro or nitroso group"
- EUR. J. MED. CHEM.--CHIM. THER. (EJMCA5,02235234);84; VOL.19 (3); PP.219-22, UNIV. BOLOGNA;IST. CHIM. FARM. TOSSICOL.; BOLOGNA; ITALY (IT), XP000676486 ANDREANI A ET AL: "Substituted 6-phenylimidazo[2,1-b]thiazoles and thiazolines as potential cardiotonic agents"
- MOLECULAR CRYSTALS AND LIQUID CRYSTALS (INC. NONLINEAR OPTICS ), Bd. 180B, Nr. PART B, 1.März 1990, Seiten 297-304, XP000237419 BARTULIN J ET AL: "SYNTHESIS AND MESOMORPHIC PROPERTIES OF 2,5-DI-(4-N-ALKYLOXYPHENYL)TH IAZOLE(5,4-D)THIAZOLES"

## Beschreibung

Die vorliegende Erfindung betrifft neue Imidazothiazol-Derivate, flüssigkristalline Mischungen, welche solche Verbindungen enthalten und die Verwendung solcher Verbindungen und Mischungen für optische und elektro-optische Vorrichtungen.

Flüssige Kristalle werden vor allem als Dielektrika in elektro-optischen Vorrichtungen, wie etwa Anzeigevorrichtungen, verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt. Das Funktionsprinzip solcher Vorrichtungen kann hierbei auf verschiedenen Effekten beruhen. So besitzen diese Vorrichtungen beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect"), OMI-Zellen ("optical mode interference") oder TFT-Zellen ("thin film transistor").

Neben den vorgenannten Zelltypen, deren Eigenschaften auf der Verwendung von nematischen oder cholesterischen Flüssigkristallen beruhen, sind in letzter Zeit Anzeigevorrichtungen bekannt geworden, welche auf dem Prinzip von gekippt ("tilted")-smektischen Phasen, insbesondere chiralen Phasen beruhen. Als gekippt-smektische Phasen eignen sich beispielsweise smektisch C, F, G, H, I und K Phasen, welche nachfolgend mit S_{C}, S_{F} usw. bezeichnet werden. Bevorzugt sind im allgemeinen S_{C} Phasen, welche besonders grosse Ansprechgeschwindigkeiten ermöglichen. Die chiral gekippten Phasen werden üblicherweise mit S_{C}*, S_{F}* usw. bezeichnet, wobei der Stern jeweils die Chiralität angibt. Bekannte Zelltypen, welche auf dem Prinzip chiraler S_{C}* Phasen beruhen sind beispielsweise SSF-Zellen (surface stabilized ferroelectric), SBF-Zellen (short-pitch bistable ferroelectric) oder DHF-Zellen (deformed helix ferroelectric).

Die Flüssigkristallmaterialien müssen eine gute chemische und thermische Beständigkeit und eine hohe Stabilität gegenüber elektrischen und magnetischen Feldern besitzen. Ferner sollten sie eine geeignete Mesophase über einen breiten Temperaturbereich, eine geringe Viskosität und kurze Ansprechzeiten besitzen. Materialien auf der Basis von chiralen gekippt-smektischen Phasen sollten ausserdem eine ausreichend hohe spontane Polarisation und je nach Zellentyp ein eher kleines (SSF-Zellen) oder ein möglichst hohes Verdrillungsvermögen (SBF-, DHF-Zellen) aufweisen. Um die Orientierung in der Zelle zu erleichtern, können diese ferner vorzugsweise eine S_{A}-Phase oberhalb der S_{C}-Phase aufweisen.

Als ferroelektrische Flüssigkristallmischungen eignen sich vorzugsweise Gemische bestehend aus mindestens einem optisch aktiven Dotierstoff und einem Flüssigkristallmaterial. Letzteres besteht aus mindestens einer, bevorzugt mehreren achiralen Komponenten, welche in der Regel eine breite getiltet-smektische Phase, vorzugsweise eine S_{C}-Phase aufweisen sollten. Die optisch aktiven Dotierstoffe müssen nicht selbst flüssigkristallin sein, können aber vorzugsweise eine smektische oder cholesterische Phase aufweisen. Die optisch aktiven Dotierstoffe sollten jedoch im Flüssigkristallmaterial eine gekippt-smektische chirale Phase mit einer geeigneten Verdrillung sowie eine ausreichend hohe spontane Polarisation induzieren.

Gegenstand der vorliegenden Erfindung sind Verbindungen der allgemeinen Formel 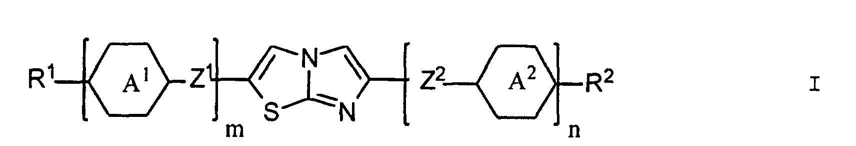 worin:
- m und n für die Zahl 0 oder 1 stehen, wobei m + n 1 oder 2 sein muss;
- R¹ eine Gruppe R³, eine cyclische chirale Gruppe Q¹ oder, falls n für die Zahl 0 und m für die Zahl 1 steht, auch eine Gruppe R³-A³-Z³-, und R² eine Gruppe R⁴, eine cyclische chirale Gruppe Q² oder, falls m für die Zahl 0 und n für die Zahl 1 steht, auch eine Gruppe R⁴-A⁴-Z⁴ bezeichnen;
- die Ringe A¹, A², A³, A⁴ trans-1,4-Cyclohexylen oder unsubstituiertes oder mit Fluor, Chlor, Cyano oder Methyl ein- oder zweifach substituiertes 1,4-Phenylen bedeuten;
- R³ und R⁴ je einen Alkyl- oder Alkenylrest mit je 3 bis 12 Kohlenstoffatomen bezeichnen, in welchem gegebenenfalls eine oder zwei nicht benachbarte Methylengruppen durch -O-, -COO- und/oder -OOC-, und/oder eines oder mehrere Wasserstoffatome durch Fluor oder Chlor ersetzt sind;
- Z¹, Z², Z³ und Z⁴ unabhängig voneinander eine Einfachbindung, CH₂-CH₂, O-CH₂, CH₂-O, COO, OOC, C≡C-, (CH₂)₄, O(CH₂)₃, CH=CH-CH₂O, OCH₂-CH=CH, CH=CH-(CH₂)₂ oder (CH₂)₂-CH=CH bezeichnen;
- und Q¹ und/oder Q² für eine der folgenden chiralen Gruppen stehen: 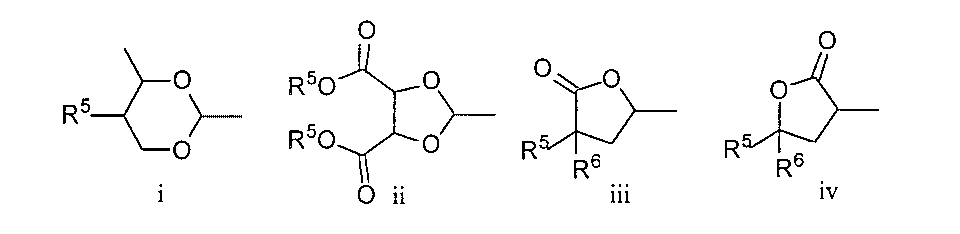 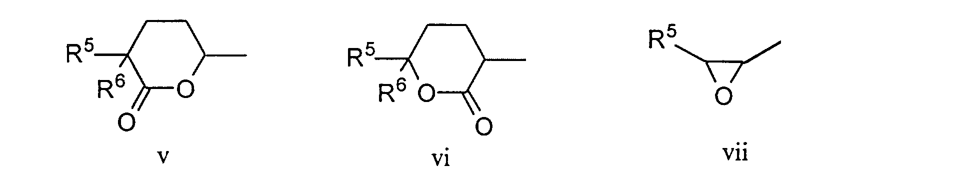
worin R⁵ und R⁶ je einen Alkyl- oder Alkenylrest darstellen, in welchem gegebenenfalls eine Methylengruppe oder zwei nicht benachbarte Methylengruppen durch -O-, -COOund/oder -OOC- und/oder eines oder mehrere Wasserstoffatome durch Fluor oder Chlor ersetzt sind, mit der Massgabe dass gegebenenfalls in R⁵ und/oder R⁶ vorhandene Sauerstoffatome nicht direkt mit dem Ring in Q¹ bzw. Q² verknüpft sind.

Es wurde überraschend gefunden, dass die Verbindungen der Formel I, welche alle eine Imidazothiazol Einheit aufweisen, eine ausgeprägte Tendenz zur Ausbildung von flüssigkristallinen Phasen, insbesondere zu gekippt-smektischen Phasen besitzen. Die optisch inaktiven Verbindungen der Formel I besitzen meist neben einer nematischen und/oder einer S_{A}-Phase eine gekippt-smektische Phase, vorallem eine S_{C}-Phase. Viele der optisch aktiven Verbindungen der Formel I besitzen neben einer cholesterischen und/oder einer S_{A}-Phase eine gekippt-smektische chirale Phase, vorallem eine S_{C}*-Phase. Diese Verbindungen sind daher besonders geeignet als Komponenten oder chirale Dotierstoffe von gekippt-mektischen chiralen Phasen in Flüssigkristallmischungen. Daneben eignen sie sich jedoch auch als Komponenten oder chirale Dotierstoffe für nematische oder cholesterische Mischungen. Die vorliegende Erfindung bietet somit eine breite Palette neuer Komponenten und Mischungen zur weiteren Optimierung und Modifizierung von Flüssigkristallmaterialien.

Die Verbindungen der Formel I besitzen eine hohe chemische Beständigkeit sowie eine hohe Stabilität gegenüber elektrischen und magnetischen Feldern. Sie sind farblos, leicht herstellbar und weisen eine ausreichende Löslichkeit untereinander und in bekannten Flüssigkristallmaterialien auf.

Die Eigenschaften der Verbindungen der Formel I können je nach Zahl und Bedeutung der Ringe und der Substituenten in breiten Bereichen variieren. Beispielsweise führen aromatische Ringe zu höheren und gesättigte Ringe zu tieferen Werten der optischen Anisotropie. Eine Erhöhung des Klärpunktes kann beispielsweise durch Einführung eines oder zwei weiterer Ringe erreicht werden. Laterale Halogensubstituenten ergeben einen Beitrag zur Dielektrizitätskonstante sowohl parallel als auch senkrecht zur Moleküllängsachse, was je nach Substitutionsmuster zur Erhöhung oder Verminderung der dielektrischen Anisotropie ausgenützt werden kann. Ferner können durch laterale Substituenten an einem oder mehreren Ringen die Mesophasenbereiche modifiziert, eine allfällige Tendenz zur Ausbildung hochgeordneter smektischer Phasen weitgehend unterdrückt und oft auch die Löslichkeit verbessert werden.

Die erfindungsgemässen Verbindungen ermöglichen daher eine weitere Optimierung der Flüssigkristallmischungen und eine Modifizierung der elektro-optischen Eigenschaften solcher Mischungen in einem weiten Bereich.

Die in den Definitionen verwendeten Ausdrücke werden im folgenden erläutert:

Der für die Definition von R3, R4, R5 und R6 verwendete Ausdruck Alkyl- oder Alkenylrest, in welchem gegebenenfalls eine oder zwei nicht benachbarte Methylengruppen durch -O-, -COO- und/oder -OOC-, und/oder ein oder mehrere Wasserstoffatome durch Fluor oder Chlor ersetzt sind" umfasst geradkettige und verzweigte (gegebenenfalls chirale) Reste wie Alkyl, 1-Alkenyl, 2-Alkenyl, 3-Alkenyl, 4-Alkenyl, Alkenyl mit endständiger Doppelbindung, Alkoxy, Alkenyloxy, Alkoxyalkyl, Alkenyloxyalkyl, Alkoxyalkenyl, Alkylcarbonyloxy, Alkylcarbonyloxyalkyl, Alkoxycarbonylalkyl, Alkoxyalkylcarbonyloxy, Fluoralkyl, Difluoralkyl, Trifluoralkyl, Chloralkyl, Cyanoalkyl, Methylalkyl, Fluoralkoxy, Fluoralkylcarbonyloxy, Methylalkoxy, Methylalkoxycarbonyl und dergleichen. Beispiele bevorzugter Reste sind Propyl, Butyl, Pentyl, Hexyl, Heptyl, Octyl, Nonyl, Decyl, Undecyl, Dodecyl, 1-Methylpropyl, 1-Methylheptyl, 2-Methylheptyl, (Z)-2-Pentenyl, (Z)-2-Hexenyl, (Z)-2-Heptenyl, (Z)-2-Octenyl, (Z)-2-Nonenyl, (E)-3-Pentenyl, (E)-3-Hexenyl, (E)-3-Heptenyl, (E)-3-Octenyl, (E)-3-Nonenyl, (Z)-4-Hexenyl, (Z)-4-Heptenyl, (Z)-4-Octenyl, (Z)-4-Nonenyl und für R5 und R6 noch zusätzlich Methyl, Ethyl; Alkenylgruppen mit endständiger Doppelbindung wie 3-Butenyl, 4-Pentenyl, 5-Hexenyl, 6-Heptenyl, 7-Octenyl, 8-Nonenyl; Propyloxy, Butyloxy, Penyloxy, Hexyloxy, Heptyloxy, Octyloxy, Nonyloxy, 1-Methylpropyloxy, 1-Methylheptyloxy, (E)-2-Propenyloxy, (E)-2-Butenyloxy, (E)-2-Pentenyloxy, (E)-2-Hexenyloxy, (E)-2- Heptenyloxy, (E)-2-Octenyloxy, (Z)-3-Pentenyloxy, (Z)-3-Hexenyloxy, (Z)-3-Heptenyloxy, (Z)-3-Octenyloxy, (Z)-3-Nonenyloxy, (E)-4-Hexenyloxy, (E)-4-Heptenyloxy, (E)-4-Octenyloxy, (E)-4-Nonenyloxy; Methoxyethyl, Methoxypropyl, Methoxybutyl, Methoxypentyl, Methoxyhexyl, Methoxyheptyl, Ethoxymethyl, Propyloxymethyl, Butyloxymethyl, Pentyloxymethyl, Hexyloxymethyl, Ethoxyethyl, Propyloxyethyl, Butyloxyethyl, Pentyloxyethyl, Hexyloxyethyl, Heptyloxyethyl, Ethoxypropyl, Propyloxypropyl, Butyloxypropyl, Pentyloxypropyl; 2-Fluorpropyloxy, 2-Fluoropentyloxy, 2-Fluoroctyloxy, 2-Chlorhexyloxy, 2-Chloroctyloxy, Butylcarbonyloxy, Pentylcarbonyloxy, Hexylcarbonyloxy, Heptylcarbonyloxy, 2-Fluorbutylcarbonyloxy, 2-Fluorpentylcarbonyloxy, 2-Fluorhexylcarbonyloxy, 2-Fluorheptylcarbonyloxy 2-Chlorbutylcarbonyloxy, 2-Chlorpentylcarbonyloxy, 2-Chlorhexylcarbonyloxy, 2-Chlorheptylcarbonyloxy, 2-Methylpentylcarbonyloxy, 2-Methylhexylcarbonyloxy, 2-Methylheptylcarbonyloxy, 2-Methylpentyloxycarbonyl, 2-Methylhexyloxycarbonyl, 2-Methylheptyloxycarbonyl, Ethyloxycarbonylmethoxy, Propyloxycarbonylmethoxy, Butyloxycarbonylmethoxy, Pentyloxycarbonylmethoxy, Hexyloxycarbonylmethoxy und für R5 und R6 noch zusätzlich Methoxy und Ethoxy.

Besonders bevorzugte Verbindungen der Formel I sind diejenigen, worin die Reste R³ bis R⁶ 3 bis 12 Kohlenstoffatome haben. Sie können geradkettig oder verzweigt sein. Die Reste R³ und R⁴ sind vorzugsweise geradkettig. Bevorzugt sind jedoch auch diejenigen Reste R³ und R⁴, welche durch eine Verzweigung und/oder durch Anwesenheit eines Fluor- oder Chlorsubstituenten zu chiralen Seitenketten führen und in den Verbindungen der Formel I zu optischer Aktivität führen. Besonders bevorzugte chirale Reste R³ und R⁴ sind 1-Methylalkyl, 1-Methylalkoxy, 1-Methylalkoxycarbonyl, 2-Fluoralkoxy, 2-Chloralkoxy, 2-Fluoralkanoyloxy und 2-Chloralkanoyloxy. Die Reste R⁵ und R⁶ sind bevorzugt geradkettig.

Z¹ bedeutet vorzugsweise eine Einfachbindung, -CH₂CH₂-, -OCH₂- oder -OOC-, Z² vorzugsweise eine Einfachbindung, -CH₂CH₂-, -CH₂O- oder -COO-; Z³ und Z⁴ bedeuten vorzugsweise eine Einfachbindung, -CH₂CH₂- -CH₂O-, -OCH₂-, -COO- oder -OOC-.

Die Ringe A¹ bis A⁴ bedeuten vorzugsweise 1,4-Phenylen, ein- bis zweifach mit Fluor substituiertes 1,4-Phenylen und/oder Cyclohexylen. Bevorzugte Fluor-substiuierte Ringe sind 2-Fluor-1,4-phenylen, 3-Fluor-1,4-phenylen und 2,3-Difluor-1,4-phenylen. Besonders bevorzugt sind im allgemeinen diejenigen Verbindungen der Formel I, in denen zumindest einer der Ringe A¹ oder A² 1,4-Phenylen, 2-Fluor-1,4-phenylen, 3-Fluor-1,4-phenylen oder 2,3-Difluor-1,4-phenylen bedeutet.

Die chiralen Ringe Q¹ und Q² kommen in den Verbindungen der Formel I vorzugsweise in einer der beiden enantiomeren Formen vor. Von den Verbindungen der Formel I, in denen Q¹ und/oder Q² vorkommen sind folglich diejenigen Verbindungen bevorzugt, in denen Q¹ und/oder Q² zu optisch aktiven Verbindungen der Formel I führen. Die Ringe Q¹ und Q² können gleich oder verschieden sein, sind aber vorzugsweise gleich. Bevorzugte Verbindungen der allgemeinen Formel I, in denen Q¹ und/oder Q² vorkommen, sind solche, in denen für Q¹ und/oder Q² die Formeln i und/oder ii stehen.

Besonders bevorzugte Untergruppen von Verbindungen der Formel I sind die Verbindungen der allgemeinen Formeln 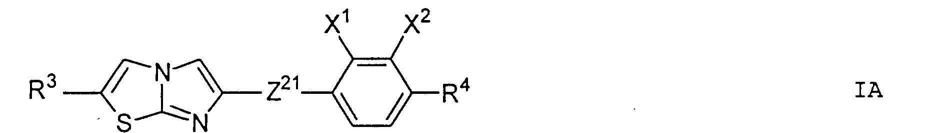 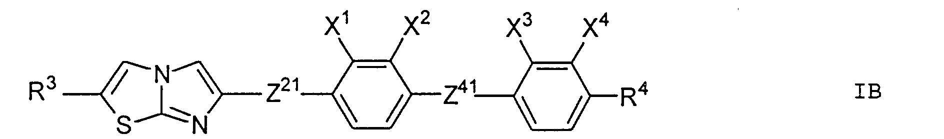 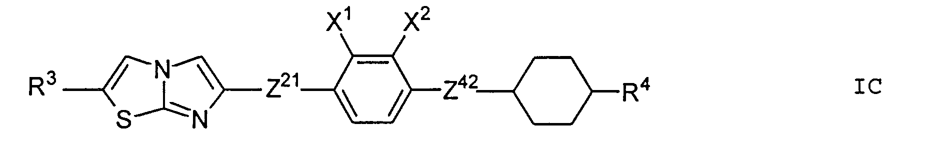 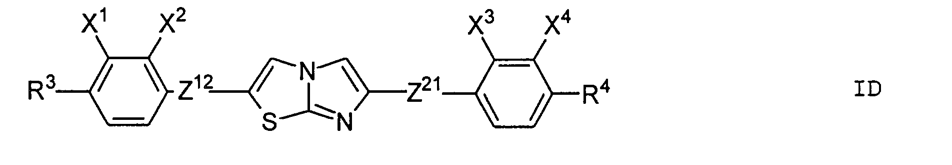 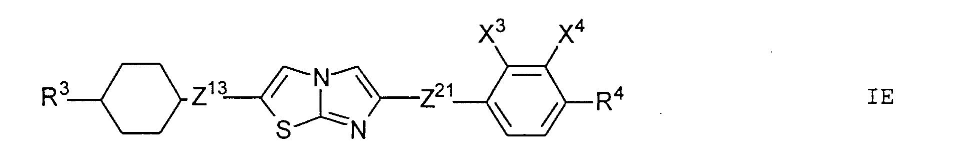 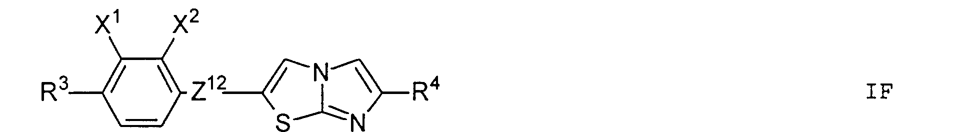 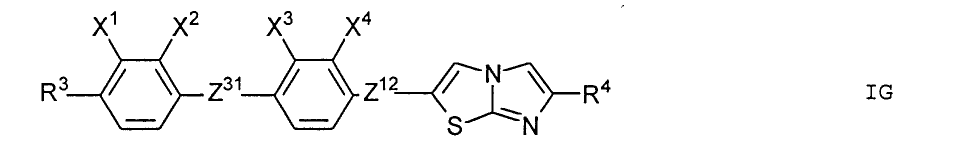 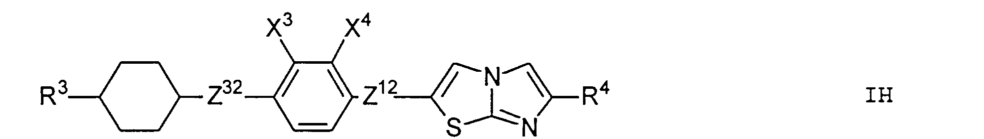 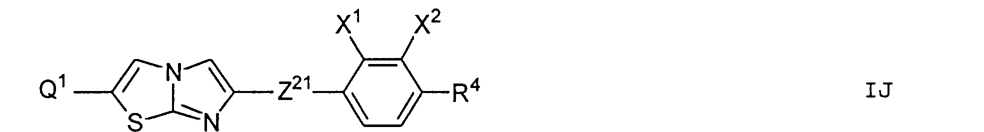 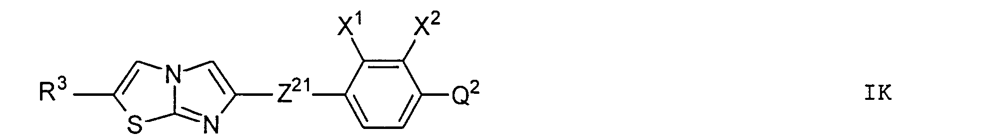 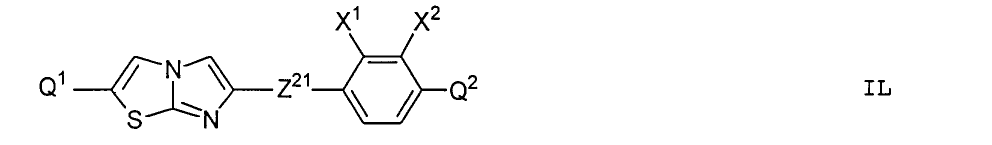 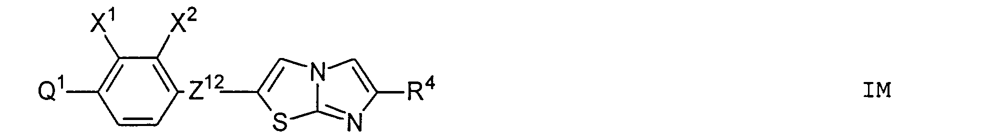 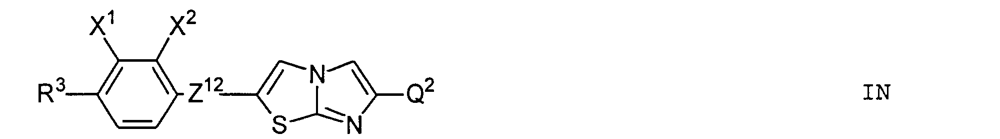 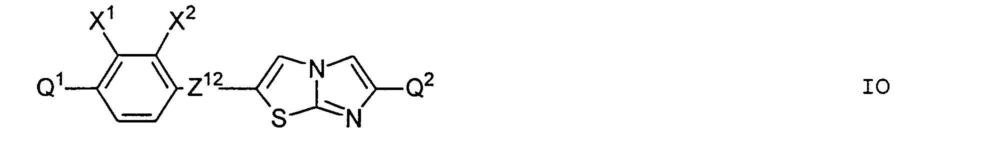 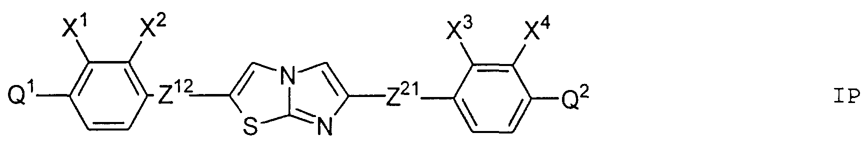 worin Q¹ und Q² sowie R³ und R⁴ die vorgenannte Bedeutung haben, Z¹², Z¹³, Z²¹, Z³¹, Z³², Z⁴¹ und Z⁴² eine Einfachbindung und/oder CH₂CH₂, Z²¹ auch CH₂O, Z¹² und Z³¹ auch OCH₂, Z⁴¹ auch CH₂O oder OCH₂, Z³² auch CH₂O oder COO und Z⁴² auch OCH₂ oder OOC bedeuten, und die Substituenten X¹ bis X⁴ für Wasserstoff oder Fluor stehen. Vorzugsweise sind jeweils höchstens einer oder zwei der Substituenten X¹ bis X⁴ von Wasserstoff verschieden und vorzugsweise höchstens eine der Gruppen Z¹² bis Z⁴² verschieden von der Einfachbindung.

Von den Verbindungen der Formeln IA bis IP sind diejenigen Verbindungen ganz besonders bevorzugt, welche ausser der Imidazothiazol Einheit und gegebenenfalls vorhandenen chiralen Ringen Q¹ und/oder Q² nur noch einen weiteren Ring aufweisen. Es sind dies die Verbindungen der Formeln IA, IF und IJ bis IO.

Die Imidazothiazole der allgemeinen Formel I können im allgemeinen gemäss nachfolgendem Schema hergestellt werden.

Dabei wird ein Aldehyd der allgemeinen Formel II zunächst mit Sulfurylchlorid und Thioharnstoff zu einem Aminothiazol der allgemeinen Formel III umgesetzt. Diese Reaktion ist aus der Literatur an analogen Beispielen beschrieben worden z.B. in Helv. Chim. Acta 38, 1291 (1955). Das Aminothiazol der Formel III wird dann analog zu J. Chem. Soc. P. I, 1989, 643 mit einem Bromaceto-Derivat der allgemeinen Formel V zum Immoniumsalz der Formel VI umgesetzt, welches ohne weitere Reinigung durch Erwärmen in einem geeigneten Lösungsmittel, wie beispielsweise Ethanol, zu I zyclisiert wird. Die Bromacetoverbindungen der Formel V sind leicht durch α-Bromierung der entsprechenden unbromierten Aceto-Derivate der allgemeinen Formel IV, beispielsweise durch Kupfer(II)bromid, erhältlich. Die Reaktion ist in J. Org. Chem. 29, 3459 (1964) an analogen Beispielen beschrieben worden.

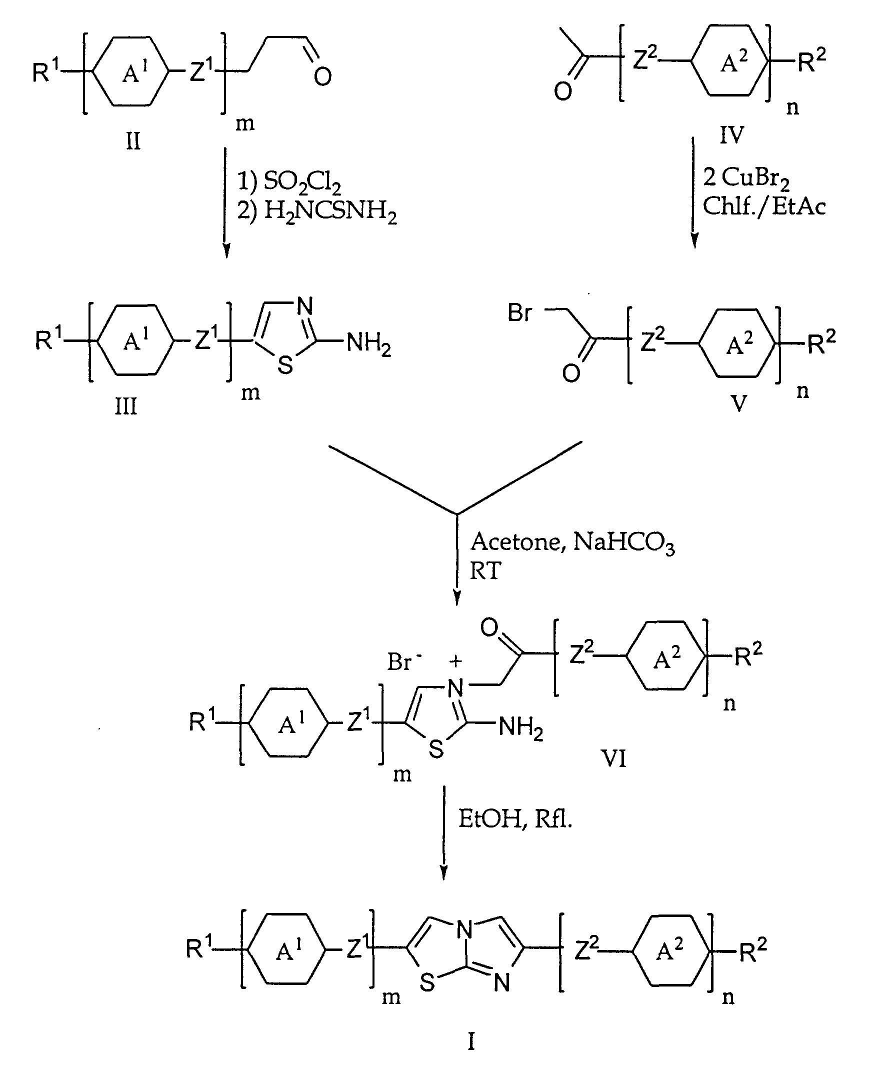

Wenn in der Gruppe R¹A¹Z¹ und/oder R²A²Z² Estergruppen vorhanden sind, kann vorzugsweise die Veresterung erst nach der beschriebenen Bildung der Imidazothiazol Einheit erfolgen. Ferner kann, wenn R¹A¹Z¹ und/oder R²A²Z² Aethergruppen aufweisen, gewünschtenfalls auch die Verätherung nach der Bildung der Imidazothiazol Einheit erfolgen.

Die zur Synthese der Verbindungen der Formel I benötigten Ausgangsmaterialien sind bekannte oder Analoge von bekannten Verbindungen oder können aus bekannten Verbindungen in wenigen Schritten hergestellt werden. Viele der Ausgangsmaterialien sind zudem im Handel erhältlich. So sind beispielsweise aus geeigneten Nitrilen durch Reduktion mit DIBAH Aldehyde herstellbar, welche bei Bedarf durch Homologisierungsreaktionen zu Aldehyden der Formel II verlängert werden können. Solche Nitrile, Aldehyde sowie Homologisierungsreaktionen sind beispielsweise in EP-A-0122389 beschrieben worden. Auch die Herstellung von Methylcarbonylen der Formel IV sind dem Fachmann im allgemeinen bekannt. So sind beispielsweise geeignete Methylketone aus entsprechenden Nitrilen durch Umsatz mit Methyllithium oder einem Methylmagnesiumhalogenid oder aus Aromaten auch durch Friedel-Crafts Acylierung leicht zugänglich. Geeignete Nitrile sind in der Flüssigkristall Literatur in grosser Zahl beschrieben worden.

Die Verbindungen der Formel I können in Form von Gemischen untereinander und/oder mit anderen Flüssigkristallkomponenten verwendet werden. Die Erfindung betrifft somit ebenfalls flüssigkristalline Mischungen mit mindestens 2 Komponenten, welche dadurch gekennzeichnet sind, dass mindestens eine Komponente eine Verbindung der Formel I ist. Eine zweite und gegebenenfalls weitere Komponenten können weitere Verbindungen der allgemeinen Formel I oder andere geeignete Flüssigkristallkomponenten sein. Bevorzugt werden die Verbindungen der Formel I als Bestandteil von Mischungen, welche eine cholesterische und/oder eine gekippt-smektische Phase, beispielsweise eine chirale S_{C}-Phase aufweisen, eingesetzt. Die Verbindungen der Formel I können dabei als achirale Komponenten und/oder als chirale Dotierstoffe verwendet werden.

Geeignete Flüssigkristallkomponenten, welche neben den Verbindungen der Formel I in den erfindungsgemässen Mischungen eingesetzt werden können, sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, oder aus Landolt-Börnstein. Liquid Crystals, Bände IV 7a-d, viele davon sind zudem im Handel erhältlich.

Aufgrund der guten Löslichkeit der erfindungsgemässen Verbindungen der Formel I in anderen Flüssigkristallmaterialien und aufgrund ihrer guten Mischbarkeit untereinander kann der Anteil an Verbindungen der Formel I in den erfindungsgemässen Mischungen relativ hoch sein und beispielsweise 0,1-50 Gew.% betragen. Werden achirale Verbindungen der Formel I als Komponenten eingesetzt, so ist im allgemeinen ein Anteil von etwa 1-40 Gew.%, insbesondere von 3-30 Gew.% bevorzugt. Der Anteil chiraler Dotierstoffe ist weitgehend durch das Verdrillungsvermögen, die spontane Polarisation und die gewünschte Ganghöhe der Mischung bestimmt. Der Anteil eines oder mehrerer gegebenenfalls eingesetzter chiraler Dotierstoffe der Formel I kann daher je nach Anwendung in einem breiten Bereich variieren und beispielsweise etwa 0,1-40 Gew.% betragen. Für Anzeigevorrichtungen auf der Basis von Flüssigkristallen mit gekippt-smektischen Phasen ist im allgemeinen ein Anteil von etwa 1-30 Gew.%, insbesondere von etwa 3-25 Gew.%, an optisch aktiven Dotierstoffen der Formel I bevorzugt.

Vorzugsweise enthalten die erfindungsgemässen Mischungen neben einer oder mehreren Verbindungen der Formel I mindestens eine weitere Verbindung aus der Gruppe von Verbindungen mit den allgemeinen Formeln 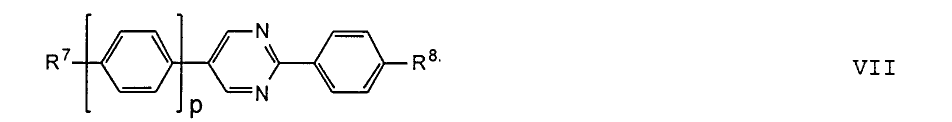 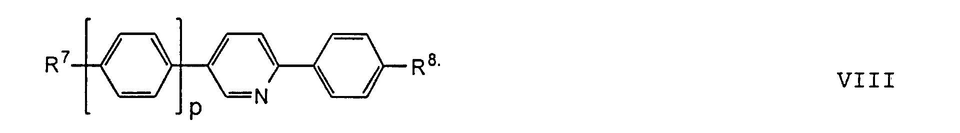 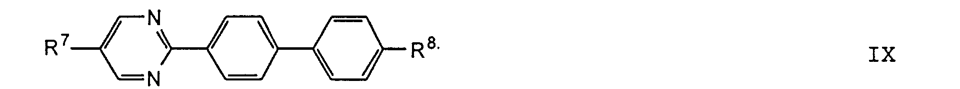 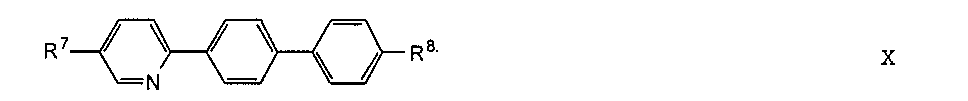 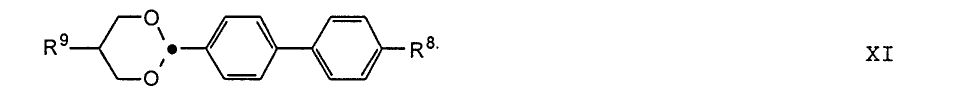 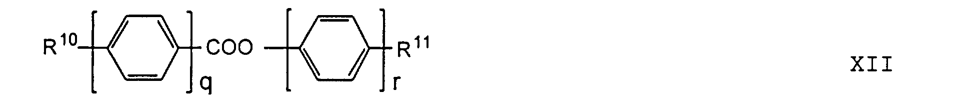 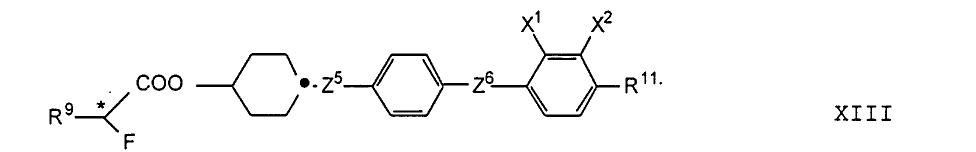 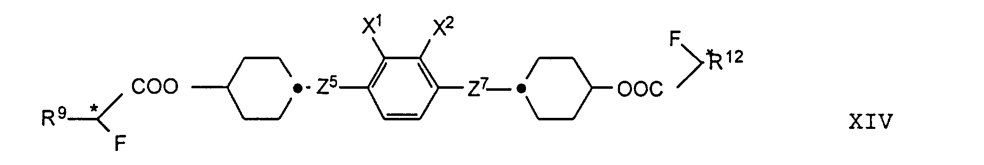 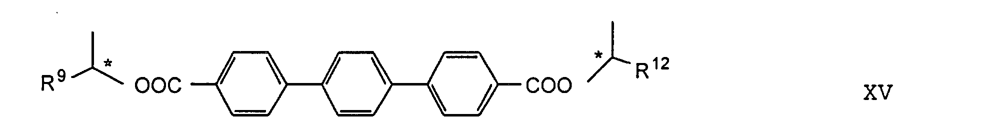 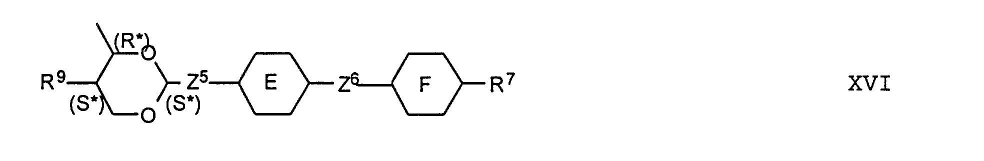 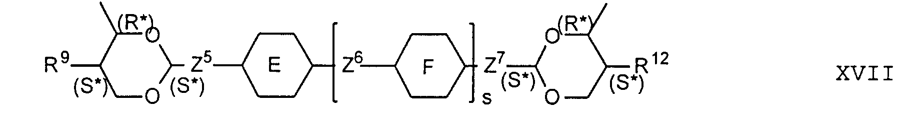 worin
- p: 0 oder 1;
- q und r: 1 oder 2, q + r = 2 oder 3;
- s: 0, 1 oder 2;
- R⁷ und R⁸: unabhängig voneinander Alkyl, Alkenyl, Alkoxy, Alkenyloxy, Alkoxyalkyl, Alkoxyalkoxy, Alkanoyloxy, Alkenoyloxy, Alkoxycarbonyl, Alkenyloxycarbonyl;
- R⁹ und R¹²: unabhängig voneinander Alkyl oder Alkenyl;
- R¹⁰ und R¹¹: unabhängig voneinander Alkyl, Alkenyl, Alkoxy und/oder Alkenyloxy;
- Z⁵, Z⁶, Z⁷: unabhängig voneinander eine Einfachbindung, CH₂-CH₂ oder CH₂O, und Z⁶ auch OCH₂, COO oder OOC;
- E und F: unabhängig voneinander Phenylen-1,4-diyl oder trans-Cyclohexylen-1,4-diyl;
- X¹ und X²: unabhängig voneinander Wasserstoff oder Fluor;
- (R*) und (S*): die relativen Konfigurationen bedeuten.

Die Verbindungen der Formeln XIII bis XVII sind optisch aktive Dotierstoffe, welche, je nach gewünschtem Effekt, neben einer oder mehreren Verbindungen der Formel I in einer der beiden enantiomeren Formen in der Mischung vorhanden sein können.

Die Substituenten R⁷ bis R¹² besitzen vorzugsweise höchstens je 18 Kohlenstoffatome; besonders bevorzugt sind etwa 5-12 Kohlenstoffatome. Die Substituenten R⁷ bis R¹² können geradlinig oder verzweigt sein, sind jedoch vorzugsweise geradlinig.

Die Herstellung von flüssigkristallinen Mischungen und elektro-optischen Vorrichtungen können in an sich bekannter Weise erfolgen.

Die Herstellung von Verbindungen der Formel I und flüssigkristallinen Mischungen der erfindungsgemässen Art werden durch die folgenden Beispiele weiter veranschaulicht. Optische Antipoden der Verbindungen der Formel I besitzen jeweils die gleichen Phasenumwandlungstemperaturen und induzieren die gleichen Absolutwerte der spontanen Polarisation und der Verdrillung aber mit entgegengesetzten Vorzeichen. Die zur Charakterisierung der Phasenumwandlungen verwendeten Abkürzungen stehen für folgende Bedeutungen:

| | |
|---|---|
| C | für kristallin, |
| S | für smektisch, |
| S_{A}, S_{B}, S_{C} etc. | für smektisch A, B, C etc., |
| S_{C}*/ S_{F}* etc. | für chiral smektisch C, F etc. |
| N | für nematisch, |
| N* | für cholesterisch, |
| I | für isotrop. |

### Beispiel 1

a) 32,1 g feingemörstertes Kupfer(II)bromid wurden mit 50 ml Essigester versetzt und während 30 Min. unter Rückfluss erhitzt. Zu dem siedenden Reaktionsgemisch wurde eine heisse Lösung von 8,3 g p-Hydroxyacetophenon in 50 ml Chloroform getropft und 5 Std. unter Rückfluss erhitzt. Das abgekühlte Gemisch wurde hierauf filtriert, der Rückstand gut mit Essigester ausgewaschen, das Filtrat mit Aktivkohle kurz zum Rückfluss erhitzt, dann abgekühlt und filtriert. Die Hauptmenge des Lösungsmittels wurde im Vakuum abgedampft und die dabei gebildeten Kristalle abgenutscht und im Hochvakuum getrocknet. Dies ergab 8,1 g 2-Brom-1-(4-hydroxyphenyl)-ethanon als hellbraune Kristalle, Smp. 122-124°C.
b) 21,3 g Nonanal wurden tropfenweise so mit 20 g Sulfurylchlorid versetzt, dass die Reaktionstemperatur zwischen 25-28°C blieb. Dann wurde die Reaktionslösung während 16 Std. bei Raumtemp. gerührt, hierauf mit 11,4 g Thioharnstoff versetzt und während 3 Std bei ca. 90°C unter Rückfluss erhitzt. Dann wurde in die noch siedende Reaktionslösung 200 ml Wasser getropft und das Reaktionsgemisch während einer weiteren Stunde bei 90°C gehalten. Danach wurde das Reaktionsgemisch heiss filtriert, abgekühlt, mit Ether extrahiert, und die abgetrennte wässrige Phase mit 2N Natronlauge auf pH 10 eingestellt. Diese wurde dann mit Ether extrahiert, die Etherphase über Natriumsulfat getrocknet, der Ether abgedampft und der feste Rückstand aus Cyclohexan umkristallisiert. Dies ergab 8 g 2-Amino-5-heptylthiazol als farblose Plättchen, Smp. 75-76°C.
c) Zu einer Lösung von 7,4 g 2-Amino-5-heptylthiazol in 30 ml trockenem Aceton wurde bei Raumtemp. eine Lösung von 7,3 g 2-Brom-1-(4-hydroxyphenyl)ethanon in 30 ml trockenem Aceton getropft. Hierauf wurde das Reaktionsgemisch während 16 Std. bei Raumtemp. gerührt und dann das Lösungsmittel im Vakuum entfernt. Der Rückstand mit einem zweiphasigen Gemisch von 300 ml Methylenchlorid und 300 ml 1M Natriumbicarbonatlösung versetzt und während 20 Min. bei Raumtemp. kräftig gerührt. Hierauf wurde der ausgefallene Feststoff abgenutscht, im Vakuum getrocknet und gleich danach in 100 ml Ethanol während 24 Std. zum Rückfluss erhitzt. Danach wurde abgekühlt, das Lösungsmittel im Vakuum abgedampft, der Rückstand in Essigester aufgenommen und das Gemisch über Kieselgel filtriert. Das Filtrat wurde hierauf eingedampft und der feste Rückstand aus Essigester/Cyclohexan umkristallisiert. Dies ergab 2,6 g 2-Heptyl-6-(4-hydroxyphenyl)imidazo-[2,1-b]thiazol als gelbliche Kristalle, Smp. 122°C.

Auf analoge Weise können die folgenden Verbindungen hergestellt werden:
- 2-Pentyl-6-(4-nonylphenyl)imidazo[2,1-b]thiazol, Smp.(C/S_{A}): 89,7°C, Klp.(S_{A}/I): 127,4°C;
- 2-Hexyl-6-(4-octylphenyl)imidazo[2,1-b]thiazol;
- 2-Hexyl-6-(4-nonylphenyl)imidazo[2,1-b]thiazol,
   Smp.(C/S_{C}): 84,1°C, S_{F}/S_{C}: 80,8°C, S_{C}/S_{A}: 96,2°C, Klp.(S_{A}/I): 124,7°C;
- 2-Hexyl-6-(4-decylphenyl)imidazo[2,1-b]thiazol;
- 2-Heptyl-6-(4-heptylphenyl)imidazo[2,1-b] thiazol;
- 2-Heptyl-6-(4-octylphenyl)imidazo[2,1-b]thiazol;
- 2-Heptyl-6-(4-nonylphenyl)imidazo[2,1-b]thiazol,
   Smp.(C/S_{C}): 86,8°C, S_{C}/S_{A}: 112,7°C, Klp.(S_{A}/I): 129°C;
- 2-Heptyl-6-(4-decylphenyl)imidazo[2,1-b]thiazol;
- 2-Octyl-6- (4-heptylphenyl)imidazo[2,1-b] thiazol;
- 2-Octyl-6-(4-octylphenyl)imidazo[2,1-b]thiazol;
- 2-Octyl-6-(4-nonylphenyl)imidazo[2,1-b]thiazol;
- 2-Octyl-6-(4-decylphenyl)imidazo[2,1-b]thiazol;
- 2-Nonyl-6-(4-heptylphenyl)imidazo[2,1-b]thiazol;
- 2-Nonyl-6-(4-octylphenyl)imidazo[2,1-b]thiazol;
- 2-Nonyl-6- (4-nonylphenyl)imidazo [2,1-b] thiazol;
- 2-Nonyl-6-(4-decylphenyl)imidazo[2,1-b]thiazol;
- 2-Decyl-6-(4-heptylphenyl)imidazo[2,1-b]thiazol;
- 2-Decyl-6-(4-octylphenyl)imidazo[2,1-b]thiazol;
- 2-Decyl-6-(4-nonylphenyl)imidazo[2,1-b]thiazol;
- 2-Heptyl-6-(2-fluor-4-nonylphenyl)imidazo[2,1-b]thiazol;
- 2-Heptyl-6-(3-fluor-4-nonylphenyl)imidazo[2,1-b]thiazol;
- 2-Heptyl-6-(2,3-difluor-4-nonylphenyl)imidazo[2,1-b]-thiazol;
- 2-Heptyl-6-[2-(4-nonylphenyl)ethyl]imidazo[2,1-b]thiazol;
- 2-Heptyl-6-(trans-4-heptylcyclohexyl)imidazo[2,1-b]-thiazol,
   Smp.(C/S_{A}): 71°C, S₁/S_{A}: 61,8°C, Klp.(S_{A}/I): 71,7°C;
- 2-Heptyl-6-[2-(trans-4-nonylcyclohexyl)ethyl]imidazo [2,1-b]thiazol;
- 2-Heptyl- 6-(4'-nonylbiphenylyl)imidazo[2,1-b]thiazol;
- 2-Heptyl- 6-(2',3'-difluor-4'-nonylbiphenylyl)imidazo-[2,1-b]thiazol;
- 2-Heptyl- 6-[4-(trans-4-nonylcyclohexyl)phenyl]imidazo-[2,1-b]thiazol;
- 2-Heptyl- 6-{4-[2-(trans-4-octylcyclohexyl)ethyl]phenyl}imidazo[2,1-b]thiazol;
- 2-(4-Heptylphenyl)-6-heptylimidazo[2,1-b]thiazol;
- 2-(4-Heptylphenyl)-6-octylimidazo[2,1-b]thiazol;
- 2-(4-Heptylphenyl)-6-nonylimidazo[2,1-b]thiazol;
- 2-(4-Heptylphenyl)-6-decylimidazo[2,1-b]thiazol;
- 2-(4-Octylphenyl)-6-heptylimidazo[2,1-b]thiazol;
- 2-(4-Octylphenyl)-6-octylimidazo[2,1-b]thiazol;
- 2-(4-Octylphenyl)-6-nonylimidazo[2,1-b]thiazol;
- 2-(4-Octylphenyl)-6-decylimidazo[2,1-b]thiazol;
- 2-(4-Nonylphenyl)-6-heptylimidazo[2,1-b]thiazol;
- 2-(4-Nonylphenyl)-6-octylimidazo[2,1-b]thiazol;
- 2-(4-Nonylphenyl)-6-nonylimidazo[2,1-b]thiazol;
- 2-(4-Decylphenyl)-6-heptylimidazo[2,1-b]thiazol;
- 2-(4-Decylphenyl)-6-octylimidazo[2,1-b]thiazol;
- 2-(2-Fluor-4-heptylphenyl)-6-nonylimidazo[2,1-b]thiazol;
- 2-(3-Fluor-4-heptylphenyl)-6-nonylimidazo[2,1-b]thiazol;
- 2-(2,3-Difluor-4-heptylphenyl)-6-nonylimidazo[2,1-b]-thiazol;
- 2-[2-(4-Heptylphenyl)ethyl]-6-nonylimidazo[2,1-b]thiazol;
- 2-(trans-4-Heptylcyclohexyl)-6-nonylimidazo[2,1-b]-thiazol;
- 2-[2-(trans-4-Heptylcyclohexyl)ethyl]-6-heptylimidazo-[2,1-b]thiazol;
- 2-(4'-Heptylbiphenylyl)-6-nonylimidazo[2,1-b]thiazol;
- 2-(2',3'-Difluor-4'-heptylbiphenyl)-6-nonylimidazo [2,1-b] thiazol;
- 2-[4-(4-trans-Heptylcyclohexyl)phenyl]-2-nonylimidazo-[2,1-b]thiazol;
- 2-{4-[2-(trans-4-Heptylcyclohexyl)ethyl]phenyl}-6-octylimidazo[2,1-b]thiazol;
- 2-(4-Nonylphenyl)-6-(4-heptylphenyl)imidazo[2,1-b]-thiazol;
- 2-(2,3-Difluor-4-nonylphenyl)-6-(4-heptylphenyl)imidazo-[2,1-b]thiazol;
- 2-(4-Nonylphenyl)-6-(2,3-difluor-4-heptylphenyl)imidazo-[2,1-b]thiazol;

### Beispiel 2

Ein Gemisch von 0,314 g 2-Heptyl-6-(4-hydroxyphenyl)-imidazo[2,1-b]thiazol (Herstellung analog Beispiel 1), 0,11 g Kaliumhydroxyd und 0,29 g Bromoctan in 15 ml Ethanol/Wasser (2:1) wurde während 16 Std. zum Rückfluss erhitzt. Dann wurde das Reaktionsgemisch abgekühlt, mit Ether extrahiert, die organische Phase über Natriumsulfat getrocknet, filtriert und eingedampft. Danach wurde der Rückstand aus abs. Ethanol umkristallisiert. Dies ergab 0,348 g 2-Heptyl-6-(4-octyloxyphenyl)imidazo[2,1-b]thiazol als farblose Kristalle,
Smp.(C/S₁): 61°C, S₁/S₂: 113°C, S₂/S₃ 115,3°C,
S₃/S_{C}: 121,7°C, Klp.(S_{C}/I): 148,9°C.

Auf analoge Weise können die folgenden Verbindungen hergestellt werden:
- 2-Heptyl-6-(4-heptyloxyphenyl)imidazo[2,1-b]thiazol;
- (R)-2-Heptyl-6-[4-(2-octyloxy)phenyl]imidazo[2,1-b]thiazol;
- (S)-2-Heptyl-6-[4-(2-octyloxy)phenyl]imidazo[2,1-b]thiazol;
- 2-Heptyl-6-[4-((E)-2-octenyloxy)phenyl]imidazo[2,1-b]-thiazol;
- 2-Heptyl-6-[4-((Z)-3-octenyloxy)phenyl]imidazo[2,1-b]-thiazol;
- 2-Heptyl-6-[4-((E)-4-octenyloxy)phenyl]imidazo[2,1-b]thiazol;
- 2-Heptyl-6-[4-(7-octenyloxy)phenyl]imidazo[2,1-b]thiazol;
- 2-Heptyl-6-(4-nonyloxyphenyl)imidazo[2,1-b]thiazol;
- 2-Hexyl-6-(4-octyloxyphenyl)imidazo[2,1-b]thiazol;
- (R)-2-Hexyl-6-[4-(2-octyloxy)phenyl]imidazo[2,1-b]thiazol;
- 2-Hexyl-6-(4-nonyloxyphenyl)imidazo[2,1-b]thiazol;
- 2-Octyl-6-(4-heptyloxyphenyl)imidazo[2,1-b]thiazol;
- 2-Octyl-6-(4-octyloxyphenyl)imidazo[2,1-b]thiazol;
   Smp.(C/S₁): 65°C, S₁/S₂: 85°C, S₂/S₃ 107°C, S₃/S_{C}: 120,5°C, Klp.(S_{C}/I): 149°C;
- (S)-2-Octyl-6-[4-(2-octyloxy)phenyl]imidazo[2,1-b]thiazol;
- 2-Octyl-6-(4-nonyloxyphenyl)imidazo[2,1-b]thiazol;
- 2-Nonyl-6-(4-heptyloxyphenyl)imidazo[2,1-b]thiazol;
- 2-Nonyl-6-(4-octyloxyphenyl)imidazo[2,1-b]thiazol;
   Smp.(C/S₁): 68°C, S₁/S₂: 99,5°C, S₂/S₃ 120°C, S₃/S_{C}: 124,5°C, Klp.(S_{C}/I): 151,5°C;
- (S)-2-Nonyl-6-[4-(2-octyloxy)phenyl]imidazo[2,1-b]thiazol;
- 2-Heptyl-6-(2-fluor-4-octyloxyphenyl)imidazo[2,1-b]thiazol;
- (S)-2-Octyl-6-[2-fluor-4-(2-octyloxy)phenyl]imidazo[2,1-b]-thiazol;
- 2-Heptyl-6-(3-fluor-4-octyloxyphenyl)imidazo[2,1-b]thiazol;
- (S)-2-Octyl-6-[3-fluor-4-(2-octyloxy)phenyl]imidazo[2,1-b]-thiazol;
- 2-Heptyl-6-(2,3-difluor-4-octyloxyphenyl)imidazo[2,1-b]-thiazol;
- (S)-2-Octyl-6-[2,3-difluor-4-(2-octyloxy)phenyl]imidazo[2,1-b]thiazol;
- 2-Heptyl-6-[2-(4-octyloxyphenyl)ethyl]imidazo[2,1-b] thiazol;
- 2-Nonyl-6-[(4-heptylphenoxy)methyl]imidazo[2,1-b]thiazol ;
- 2-Octyl-6-(4'-octyloxybiphenyl)imidazo[2,1-b]thiazol;
- 2-Octyl-6-(2',3'-difluor-4'-octyloxybiphenyl)imidazo[2,1-b]thiazol;
- (S)-2-Octyl-6-[2',3'-difluor-4'-(2-octyloxy)biphenyl]-imidazo[2,1-b]thiazol ;
- 2-Heptyl-6-{4-[(trans-4-octylcyclohexyl]methoxy]phenyl} imidazo [2,1-b] thiazol;
- 2-(4-Heptyloxyphenyl)-6-heptylimidazo[2,1-b]thiazol;
- 2-(4-Heptyloxyphenyl)-6-octylimidazo[2,1-b]thiazol;
- 2-(4-Heptyloxyphenyl)-6-nonylimidazo[2,1-b]thiazol;
- 2-(4-Heptyloxyphenyl)-6-decylimidazo[2,1-b]thiazol;
- 2-(4-Octyloxyphenyl)-6-heptylimidazo[2,1-b]thiazol;
- 2-(4-Octyloxyphenyl)-6-octylimidazo[2,1-b]thiazol;
- (S)-2-[4-(2-Octyloxy)phenyl]-6-octylimidazo[2,1-b]thiazol;
- 2-[4-((E)-2-Octenyloxy)phenyl]-6-heptylimidazo[2,1-b]thiazol;
- 2-[4-((Z)-3-Octenyloxy)phenyl]-6-heptyl-imidazo[2,1-b]-thiazol;
- 2-[4-((E)-4-Octenyloxy)phenyl]-6-heptylimidazo[2,1-b]-thiazol;
- 2-(4-Nonyloxyphenyl)-6-heptylimidazo[2,1-b]thiazol;
- 2-(4-Nonyloxyphenyl)-6-octylimidazo[2,1-b]thiazol;
- 2-(2-Fluor-4-octyloxyphenyl)-6-octylimidazo[2,1-b]thiazol;
- 2-(4-Nonyloxyphenyl)-6-octylimidazo[2,1-b]thiazol;
- (S)-2-[3-Fluor-4-(2-octyloxy)phenyl]-6-octylimidazo[2,1-b]-thiazol;
- 2-(2,3-Difluor-4-octyloxyphenylphenyl)-6-octylimidazo[2,1-b]thiazol;
- 2-[2-(4-Heptylphenyl) ethyl]-6-nonylimidazo [2,1-b] thiazol;
- 2-(trans-4-Heptylcyclohexyl)-6-nonylimidazo[2,1-b]thiazol;
- 2-[2-(trans-4-Heptylcyclohexyl)ethyl]-6-heptylimidazo[2,1-b]thiazol;
- 2-(4'-Heptylbiphenylyl)-6-nonylimidazo[2,1-b]thiazol;
- 2-(2',3'-Difluor-4'-heptylbiphenylyl)-6-nonylimidazo [2,1-b]thiazol;
- 2-{4-[(trans-4-Octylcyclohexyl)methoxy]phenyl}-2-heptylimidazo[2,1-b]thiazol;
- 2-(4-Octylphenyl)-6-(4-octyloxyphenyl)imidazo[2,1-b]thiazol;
- 2-(4-Octyloxyphenyl)-6-(4-octylphenyl)imidazo[2,1-b]thiazol;
- 2-(2,3-Difluor-4-octyloxyphenyl)-6-(4-octylphenyl)imidazo-[2,1-b]thiazol;
- 2-(4-Octyloxyphenyl)-6-(2,3-difluor-4-octylphenyl)imidazo-[2,1-b]thiazol;
- 2-(4-Octyloxyphenyl)-6-(2,3-difluor-4-octyloxyphenyl)-imidazo[2,1-b]thiazol;
- 2-(2,3-Difluor-4-octyloxyphenyl)-6-(4-octyloxyphenyl)-imidazo[2,1-b]thiazol;
- (S,S)-2-[4-(2-Octyloxy)phenyl]-6-[4-(2-octyloxy)phenyl]-imidazo[2,1-b]thiazol;

### Beispiel 3

a) Ein Gemisch von 2 g 2-Heptylimidazo[2,1-b]thiazol-6-carbonsäureethylester, hergestellt aus α-Brombrenztraubensäureethylester analog zu Beispiel 1, in 50 ml 1N methanolischer KOH wird über Nacht bei Raumtemp. stehen gelassen, dann mit 1N Schwefelsäure angesäuert, mit 200 ml Wasser verdünnt und dann mit Ether extrahiert. Die Etherphase wird hierauf über Natriumsulfat getrocknet, filtriert und eingedampft. Dies ergibt 2-Heptylimidazo[2,1-b]thiazol-6-carbonsäure.
b) Zu einer Lösung von 1,3 g 2-Heptylimidazo[2,1-b]-thiazol-6-carbonsäure, 1 g 4-Octyloxyphenol und 0,1 g 4-(Dimethylamino)pyridin in 25 ml Dichlormethan werden innert 10 Min. unter Rühren portionenweise 1,2 g N,N'-Dicyclohexylcarbodiimid gegeben. Das Gemisch wird über Nacht bei Raumtemperatur gerührt und dann filtriert. Das Filtrat wird mit Dichlormethan verdünnt, zweimal mit je 50 ml gesättigter Natriumcarbonat-Lösung und dann mit Wasser gewaschen, über Magnesiumsulfat getrocknet, filtriert und das Filtrat eingedampft. Umkristallisation des Rückstandes aus Essigester/Cyclohexan ergibt 2-Heptylimidazo[2,1-b]thiazol-6-carbonsäure-4-octyloxyphenylester, Smp.(C/S_{A}): 112,6°C, Kip.(S_{A}/I): 151,4°C.

Auf analoge Weise können hergestellt werden:
- (S)-2-Heptylimidazo[2,1-b]thiazol-6-carbonsäure-4-(2-octyloxy)phenylester;
- 2-Heptylimidazo[2,1-b]thiazol-6-carbonsäure-4-octylphenylester;
- 2-(4-Octylphenyl)imidazo[2,1-b]thiazol-6-carbonsäureoctylester;
- 2-(4-Octyloxyphenyl)imidazo[2,1-b]bhiazol-6-carbonsäureoctylester;
- (S)-2-(4-Octyloxyphenyl)imidazo[2,1-b]thiazol-6-carbonsäure(2-octyl)ester;
- 2-[4-((S)-2-Octyloxy)phenyl]imidazo[2,1-b]thiazol-6-carbonsäure((S)2-octyl)ester;
- 2-[4-((S)-2-Octyloxy)phenyl]imidazo[2,1-b]thiazol-6-carbonsäure((R)2-octyl)ester;
- 4-(2-Heptylimidazo[2,1-b]thiazol-6-yl)benzoesäureoctylester, Smp.(C/S₁): 61.8°C, S₁/S_{A}: 97°C, Klp.(S_{A}/I): 129°C;
- (S)4-(2-Octylimidazo[2,1-b]thiazol-6-yl)benzoesäure-2-octylester;
- 4-(6-Heptylimidazo[2,1-b] thiazol-2-yl)benzoesäureoctylester;
- (R)4-(6-Heptylimidazo[2,1-b]thiazol-2-yl)benzoesäure-2-octylester;Smp.(C/I): 112,4°C;
- (S)4-(6-Octylimidazo[2,1-b]thiazol-2-yl)benzoesäure-2-octylester;
- (S,S)-2-[4- (2-Octyloxcarbonyl)phenyl]imidazo[2,1-b]thiazol-6-carbonsäure(2-octyl)ester;
- (S,S)-6-[4-(2-Octyloxcarbonyl)phenyl]imidazo[2,1-b] thiazol-2-carbonsäure(2-octyl)ester,
   Smp.(C/N*): 62,7°C, Klp.(N*/I): 66,2°C;
- Nonancarbonsäure-4-(2-heptylimidazo[2,1-b]thiazol-6-yl)-phenylester,
   Smp.(C/S_{C}): 92,7°C, S_{C}/S_{A}: 119°C, Klp.(S_{A}/I): 147°C;
- Octancarbonsäure-4-(2-octylimidazo[2,1-b]thiazol-6-yl)-phenylester;
- Octancarbonsäure-4-(2-nonylimidazo[2,1-b] thiazol-6-yl)-phenylester;
- Nonancarbonsäure-4-(2-octylimidazo[2,1-b]thiazol-6-yl)-phenylester;
- Decancarbonsäure-2,3-difluor-4-(2-heptylimidazo[2,1-b]thiazol-6-yl)phenylester, Smp.(C/N): 85,1°C,
   N/S_{C}: 90,2°C, Klp.(S_{C}/I): 96,6°C;
- trans-4-heptylcyclohexancarbonsäure-4-(2-octylimidazo[2,1-b]thiazol-6-yl)phenylester;
- Nonancarbonsäure-4-[2-(4-octylphenyl)imidazo[2,1-b]thiazol-6-yl]phenylester;
- (R)-2-Fluorhexancarbonsäure-4-(2-octylimidazo[2,1-b]-thiazol-6-yl)phenylester;
- (R)-2-Fluorhexancarbonsäure-2,3-difluor-4-(2-octylimidazo-[2,1-b]thiazol-6-yl)phenylester, Smp.(C/ S_{A}): 88,3°C, Klp.(S_{A}/I): 91,8°C;
- (R)-2-Chlorhexancarbonsäure-4-(2-octylimidazo[2,1-b]-thiazol-6-yl)phenylester;
- Heptancarbonsäure-4-(6-nonylimidazo[2,1-b]thiazol-2-yl)-phenylester;
- Octancarbonsäure-4-(6-octylimidazo[2,1-b]thiazol-2-yl)-phenylester;
- Nonancarbonsäure-4-(6-heptylimidazo[2,1-b]thiazol-2-yl)-phenylester;
- (R)-2-Fluorhexancarbonsäure-4-(6-nonylimidazo[2,1-b]-thiazol-2-yl)phenylester;
- (R)-2-Chlorhexancarbonsäure-4-(6-octylimidazo[2,1-b]-thiazol-2-yl)phenylester;
- trans-4-Heptylcyclohexancarbonsäure-4-(6-octylimidazo[2,1-b]thiazol-2-yl)phenylester;
- Nonancarbonsäure-4-[6-(4-octylphenyl)imidazo[2,1-b]thiazol-2-yl]phenylester;

### Beispiel 4

Ein Gemisch von 0,08 g 4-(2-Carboxyimidazo[2,1-b]thiazol-6-yl)benzaldehyd, 0,133 g (2R,3S)-2-Octyl-1,3-butandiol (Herstellung: siehe EP-A-0 457 105), 15 ml Toluol und 3 Tropfen 2n Schwefelsäure wurde unter Wasserabscheidung während 2 Stunden bei Rückfluss gekocht. Dann wurde abgekühlt, 1 ml Triethylamin zugegeben und zwischen Ether und gesättigter Natriumbicarbonatlösung verteilt. Die organische Lösung wurde hierauf über Magnesiumsulfat getrocknet, filtriert, zur Trockne eingedampft und der Rückstand an Kieselgel mit Toluol/Essigester (10:1) chromatographiert. Anschliessende Kristallisation aus Essigester/Hexan (1:3) ergab 0,079 g 2-((2R, 3S, 5R)-4-Metyl-5-octyl-1,3-dioxan-2-yl)-6-[4-((2R, 3S, 5R)-4-Methyl-5-octyl-1,3-dioxan-2-yl)-phenyl]imidazo[2,1-b]thiazol, Smp. (C/S_{A}) : 179°C, Klp.(S_{A}/I): 191,2°C.

Auf analoge Weise können hergestellt werden:
- 2-((2S, 3R, 5S)-4-Methyl-5-octyl-1,3-dioxan-2-yl)-6-[4-((2S, 3R, 5S)-4-Methyl-5-octyl-1,3-dioxan-2-yl)-phenyl]imidazo[2,1-b]thiazol;
- 2-((2R, 3S, 5R)-4-Methyl-5-hexyl-1,3-dioxan-2-yl)-6-[4-((2R, 3S, 5R)-4-Methyl-5-hexyl-1,3-dioxan-2-yl)-phenyl]imidazo[2,1-b]thiazol;
- 2-((2R, 3S, 5R)-4-Methyl-5-decyl-1,3-dioxan-2-yl)-6-[4-((2R, 3S, 5R)-4-Methyl-5-decyl-1,3-dioxan-2-yl)-phenyl]imidazo[2,1-b]thiazol;
- 2-((4R, 5R)-4,5-Dimethoxycarbonyl-1,3-dioxolanyl)-6-[4-((4R, 5R)-4,5-Dimethoxycarbonyl-1,3-dioxolanyl)]-imidazo[2,1-b]thiazol;
- 2-((4S, 5S)-4,5-Dimethoxycarbonyl-1,3-dioxolanyl)-6-[4-((4S, 5S)-4,5-Dimethoxycarbonyl-1,3-dioxolanyl)]-imidazo[2,1-b]thiazol;
- 2-((4R, 5R)-4,5-Diethoxycarbonyl-1,3-dioxolanyl)-6-[4-((4R, 5R)-4,5-Diethoxycarbonyl-1,3-dioxolanyl)]-imidazo [2,1-b]thiazol;

### Beispiel 5

Zur Untersuchung der Eigenschaften der Verbindungen der Formel I in Gemischen wurden Testmischungen hergestellt. Dazu wurde die zu testende Komponente der Formel I mit einer Grundmischung (GM) vermischt. Zu Vergleichszwecken wurde zudem eine Vergleichsmischung hergestellt, wobei zur Grundmischung GM eine seiner Komponenten als Vergleichskomponente zugemischt wurde. Von diesen Mischungen wurde die Phasenfolge bestimmt sowie die spontane Porarisation (Pₛ in nC/cm²), die Schaltzeit und der Schaltwinkel gemessen. Die Messungen wurden unter den folgenden Bedingungen durchgeführt: Pₛ bei 8,5 µ Zelldicke und einer Dreieckspannung von 10 Hz und 5 V/µ; Schaltzeiten bei 10 V/µ Rechteckspannung (Pik/Pik) bestimmt als Zeit bis Iₘₐₓ und Schaltwinkel bei 2µ-Zelldicke und einer Spannung von 25V. Für alle Messungen wurde eine Temperatur von 25°C eingehalten.

### Grundmischung (GM)

| | |
|---|---|
| 16,6 Gew.% | trans-4-[4-(2,3-Difluoro-4-octyloxybenzoyloxy)phenyl]cyclohexyl(R)-2-fluorhexanoat; |
| 23,8 Gew.% | 2-(4-Hexyloxyphenyl)-5-nonylpyrimidin; |
| 23,4 Gew.% | 2-(4-Nonyloxyphenyl)-5-nonylpyrimidin; |
| 11,8 Gew.% | 2-(4-Nonyloxyphenyl)-5-octylpyrimidin; |
| 12,1 Gew.% | 2-(4-Heptyloxyphenyl)-5-heptylpyrimidin; |
| 12,3 Gew.% | 2-(4-Decyloxyphenyl)-5-octylpyrimidin; |

Phasenfolge [°C]:
I - 74,6 - N* - 67,7 - S_{A} - 60,6-S_{C}*- -7,6 C

### Vergleichsmischung

| | |
|---|---|
| 15,0 Gew.% | 2-(4-Nonyloxyphenyl)-5-nonylpyrimidin; |
| 85,0 Gew.% | GM; |

Phasenfolge[°C]:
I - 71,0 - N*- 66,0- S_{A}- 60,0 - S_{C}* - ;
Ps: 16,0 nC/cm², Schaltzeit: 120 µs, Schaltwinkel: 50,2°.

### Testmischung 1

| | |
|---|---|
| 15,0 Gew.% | 2-Heptyl-6-(4-nonylphenyl)imidazo[2,1-b]-thiazol; |
| 85,0 Gew.% | GM; |

Phasenfolge [°C]:
I - 76,7 - N* - 74,5 - S_{A} - 61,7 - S_{C}* - ;
Ps: 17,7 nC/cm², Schaltzeit: 100 µs, Schaltwinkel: 40°

### Testmischung 2

| | |
|---|---|
| 15,0 Gew.% | 2-Hexyl-6-(4-nonylphenyl)imidazo[2,1-b]thiazol; |
| 85,0 Gew.% | GM; |

Phasenfolge:
I - 74,4 N* - 71,4 S_{A}- 60,3 - S_{C}* - ;
Ps: 16,8 nC/cm², Schaltzeit: 132 µs, Schaltwinkel: 52,1°.

### Testmischung 3

| | |
|---|---|
| 15,0 Gew.% | 2-Pentyl-6-(4-nonylphenyl)imidazo[2,1-b]-thiazol; |
| 85,0 Gew.% | GM; |

Phasenfolge [°C]:
I - 76,1 N* - 73,0 S_{A}- 59,9 S_{C}* - ;
Ps: 16,1 nC/cm², Schaltzeit: 132 µs, Schaltwinkel: 51,3°.

### Testmischung 4

| | |
|---|---|
| 15,0 Gew.% | 2-Heptyl-6-(4-octyloxyphenyl)imidazo[2,1-b]thiazol; |
| 85,0 Gew.% | GM; |

Phasenfolge [°C]:
I - 80,9 N* - 75,3 S_{A}- 70,6 - S_{C}* - ;
Ps: 20,1 nC/cm², Schaltzeit: 150 µs, Schaltwinkel: 60,3°.

### Testmischung 5

| | |
|---|---|
| 15,0 Gew.% | Nonancarbonsäure-4-(2-heptylimidazo[2,1-b]-thiazol-6-yl)phenylester; |
| 85,0 Gew.% | GM; |

Phasenfolge [°C]
I - 78,7 - N* - 73,6 - S_{A} - 68,2 - S_{C}* - ;
Ps: 20,5 nC/cm², Schaltzeit: 140 µs, Schaltwinkel: 56,2°.

### Testmischung 6

| | |
|---|---|
| 15,0 Gew.% | 2-Heptylimidazo[2,1-b]thiazol-6-carbonsäure-4-octyloxyphenylester; |
| 85,0 Gew.% | GM; |

Phasenfolge [°C]:
I - 78,2 - N* - 71,5 - S_{A} - 61,3 - S_{C}* - ;
Ps: 16,3 nC/cm².

## Patentansprüche

1. Imidazothiazol-Derivate der allgemeinen Formel 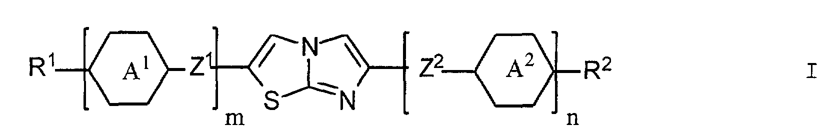 in der die Symbole m, n, R¹ und R² folgende Bedeutung haben:
m und n 0 oder 1, wobei m + n = 1 oder 2,
R¹ R³ oder Q¹ oder, falls n = 0 und m = 1 auch R³-A³-Z³-,
R² R⁴ oder Q² oder, falls n = 1 und m = 0 auch R⁴-A⁴-Z⁴-,
wobei unabhängig voneinander
- R³ und R⁴ je einen Alkyl- oder Alkenylrest mit je drei bis 12 Kohlenstoffatomen darstellen, in welchem gegebenenfalls eine Methylengruppe oder mehrere nicht benachbarte Methylgruppen durch -O-, -COO-, oder -OOC- und/oder ein Wasserstoffatom oder mehrere Wasserstoffatome durch Fluor oder Chlor ersetzt sind,
- Q¹ und Q² für je eine der folgenden chiralen Gruppen stehen 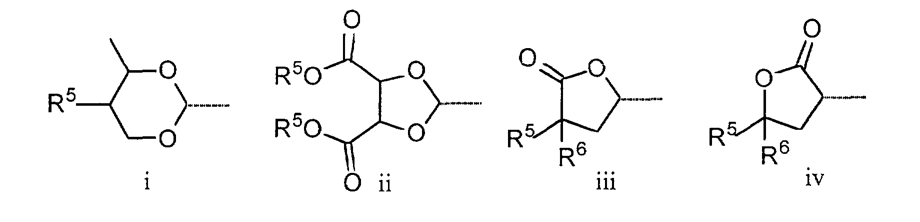 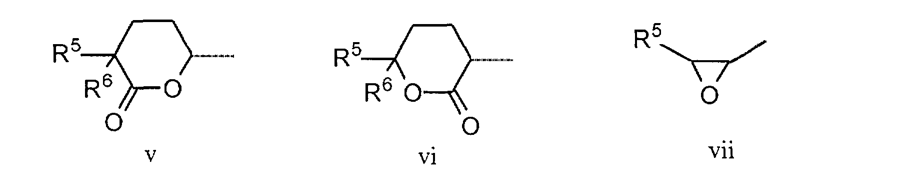
- worin R⁵ und R⁶ je einen Alkyl- oder Alkenylrest darstellen, in welchem eine Methylengruppe oder mehrere nicht benachbarte Methylengruppen - mit der Massgabe, dass die Sauerstoffatome nicht direkt mit dem Ring in Q¹ oder Q² verknüpft sind - durch -O-, -COO- oder -OOCund/oder ein Wasserstoffatom oder mehrere Wasserstoffatome durch Fluor oder Chlor ersetzt sein können,
- A¹, A², A³ und A⁴ für trans-1,4-Cyclohexylen oder unsubstituiertes oder mit Fluor, Chlor, Cyano oder Methyl ein- oder zweifach substituiertes 1,4-Phenylen stehen, und
- Z¹, Z², Z³ und Z⁴ je eine Einfachbindung, -CH₂-CH₂-, -O-CH₂-, -CH₂-O-, -COO-, -OOC-, -C≡C-, -(CH₂)₄-, -O(CH₂)₃-, -CH=CH-CH₂O-, -OCH₂-CH=CH-, -CH=CH-(CH₂)₂- oder -(CH₂)₂-CH=CH- bezeichnen.

2. Imidazothiazol-Derivate nach Anspruch 1, **dadurch gekennzeichnet, dass** es optisch inaktiv ist und eine gekippt-smektische Phase ausbildet.

3. Imidazothiazol-Derivate nach Anspruch 1, **dadurch gekennzeichnet, dass** es optisch aktiv ist.

4. Imidazothiazol-Derivate nach einem der Ansprüche 1 bis 3, **dadurch gekennzeichnet, dass** die Reste R⁵ und R⁶ geradkettig oder verzweigt sind und je 3 bis 12 Kohlenstoffatome aufweisen.

5. Imidazothiazol-Derivate nach einem der Ansprüche 1 bis 4, **dadurch gekennzeichnet, dass** A¹ und/oder A² 1,4 Phenylen, 2-Fluor-1,4-Phenylen, 3-Fluor-1,4-Phenylen oder 2,3-Difluor-1,4-phenylen bedeutet.

6. Imidazothiazol-Derivate nach einem der Ansprüche 1 bis 5, **dadurch gekennzeichnet, dass** für Q¹ und/oder Q² die allgemeine Formel i und/oder ii steht.

7. Flüssigkristalline Mischungen, bestehend aus mindestens zwei Komponenten, **gekennzeichnet durch** einen Gehalt an mindestens einem Imidazothiazol-Derivat der allgemeinen Formel I.

8. Flüssigkristalline Mischung nach Anspruch 7, bestehend aus mindestens einem optisch aktiven Dotierstoff und einem Flüssigkristallmaterial mit mindestens einer achiralen Komponente, **dadurch gekennzeichnet, dass** der mindestens eine Dotierstoff aus einem chiralen Imidazothiazol-Derivat der allgemeinen Formel I und/oder die mindestens eine Komponente aus einem achiralen Imidazothiazol-Derivat der allgemeinen Formel I gebildet wird.

9. Flüssigkristalline Mischung nach Anspruch 7 oder 8, **dadurch gekennzeichnet, dass** sie mindestens eine Verbindung aus der Gruppe von Verbindungen mit den allgemeinen Formeln 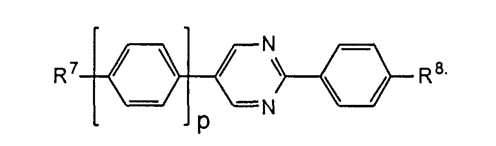 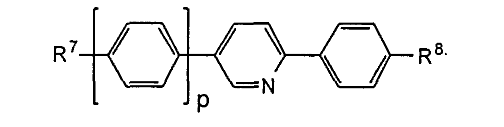 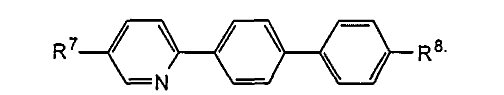 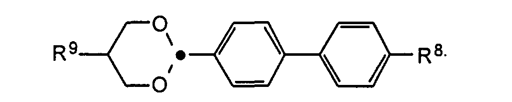 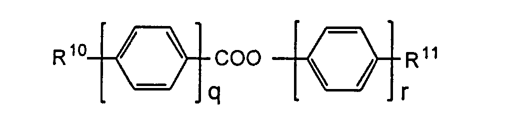 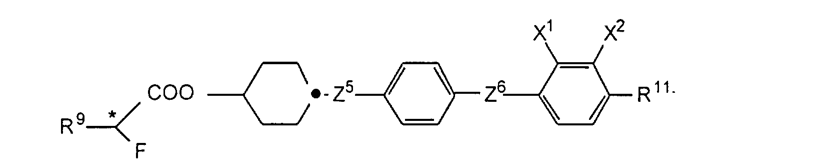 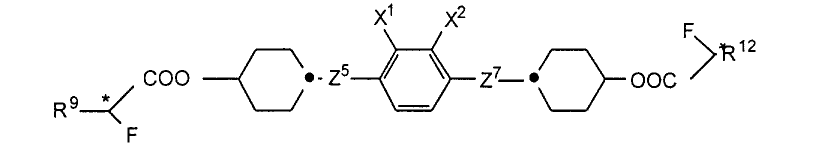 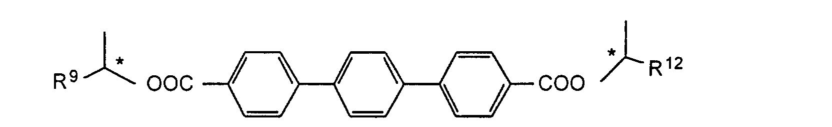 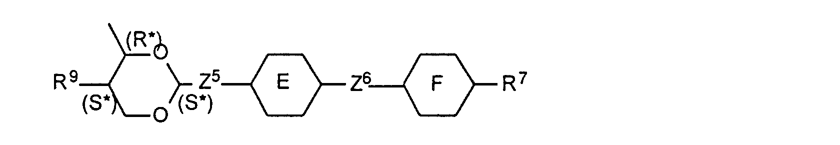 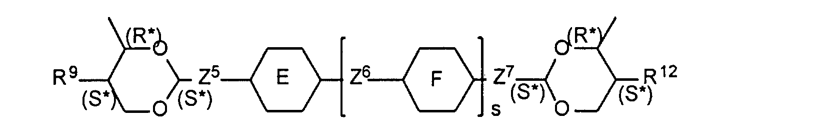 enthält, worin
p 0 oder 1;
q und r 1 oder 2, q + r = 2 oder 3;
s 0, 1 oder 2;
R⁷ und R⁸ unabhängig voneinander Alkyl, Alkenyl, Alkoxy, Alkenyloxy, Alkoxyalkyl, Alkoxyalkoxy, Alkanoyloxy, Alkenoyloxy, Alkoxycarbonyl, Alkenyloxycarbonyl;
R⁹ und R¹² unabhängig voneinander Alkyl oder Alkenyl;
R¹⁰ und R¹¹ unabhängig voneinander Alkyl, Alkenyl, Alkoxy und/oder Alkenyloxy;
Z⁵, Z⁶, Z⁷ unabhängig voneinander eine Einfachbindung, CH₂-CH₂ oder CH₂O, und Z⁶ auch OCH₂, COO oder OOC;
E und F unabhängig voneinander Phenylen-1,4-diyl oder trans-Cyclohexylen-1,4-diyl;
X¹ und X² unabhängig voneinander Wasserstoff oder Fluor;
(R*) und (S*) die relativen Konfigurationen bedeuten.

10. Optische oder elektrooptische Vorrichtungen enthaltend mindestens ein Imidazothiazol-Derivat der allgemeinen Formel I.

11. Optische oder elektrooptische Vorrichtungen enthaltend eine flüssigkristalline Mischung nach einem der Ansprüche 7, 8 oder 9.

## Claims

1. An imidazothiazole derivative of the formula 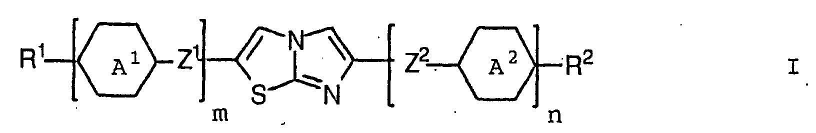 in which the symbols m, n, R¹ and R² have the following meanings:
m and n are 0 or 1, whereby m and n is 1 or 2
R¹ is R³ or Q¹ or, if n is 0 and m is 1, also R³-A³-Z³-,
R² is R⁴ or Q² or, if n is 1 and m is 0, also R⁴-A⁴-Z⁴-,
and, independently of one another,
- R³ and R⁴ are each an alkyl or alkenyl radical having each 3 to 12 carbon atoms in which one methylene group or a plurality of non-neighbouring methylene groups are optionally replaced by -O-, -COO- or -OOC-, and/or one hydrogen atom or a plurality of hydrogen atoms are replaced by fluorine or chlorine
- Q¹ and Q² are each one of the following chiral groups 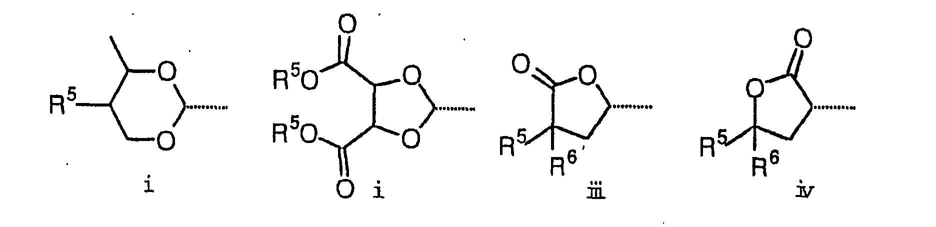 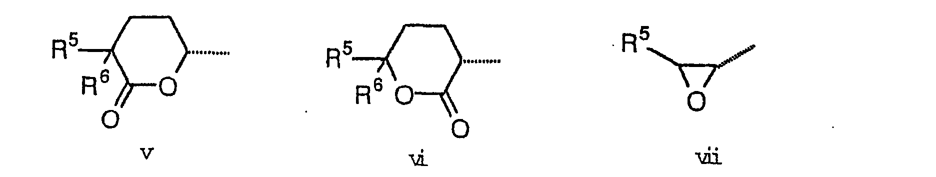
- in which R⁵ and R⁶ are each an alkyl or alkenyl radical in which one methylene group or a plurality of non-neighbouring methylene groups may be replaced by -O-, -COO- or -OOC-, and/or one hydrogen atom or a plurality of hydrogen atoms may be replaced by fluorine or - chlorine, with the proviso that the oxygen atoms are not linked directly to the ring in Q¹ or Q²,
- A¹, A², A³ and A⁴ are trans-1,4-cyclohexylene or are 1,4-phenylene which is unsubstituted or monosubstituted or disubstituted by fluorine, chlorine, cyano or methyl, and
- Z¹, Z², Z³ and Z⁴ are each a single bond, -CH₂-CH₂-, -O-CH₂-, -CH₂-O-, -COO-, -OOC-, -C≡C-, -(CH₂)₄-, -O(CH₂)₃-, -CH=CH-CH₂O-, -OCH₂-CH=CH-, -CH=CH-(CH₂)₂- or - (CH₂)₂-CH=CH-.

2. An imidazothiazole derivative as claimed in claim 1, which is optically inactive and forms a tilted smectic phase

3. An imidazothiazole derivative as claimed in claim 1, which is optically active.

4. An imidazothiazole derivative as claimed in any of claims 1 to 3, wherein the radicals R⁵ and R⁶ are straight-chain or branched and each have 3 to 12 carbon atoms.

5. An imidazothiazole derivative as claimed in any of claims 1 to 4, wherein A¹ and/or A² are 1,4-phenylene, 2-fluoro-1,4-phenylene, 3-fluoro-1,4-phenylene or 2,3-difluoro-1,4-phenylene.

6. An imidazothiazole derivative as claimed in any of claims 1 to 5, wherein Q¹ and/or Q² are of the formulae i and/or ii.

7. A liquid crystalline mixture having at least two components, comprising a content of at least one imidazothiazole derivative of the formula I.

8. A liquid crystalline mixture as claimed in claim 7, having at least one optically active dopant and a liquid crystalline material with at least one achiral component, wherein the one or more dopants are formed from a chiral imidazothiazole derivative of the formula I and/or the one or more components are formed from an achiral imidazothiazole derivative of the formula I.

9. A liquid crystalline mixture as claimed in claim 7 or 8, which contains at least one compound from the group consisting of compounds of the formulae 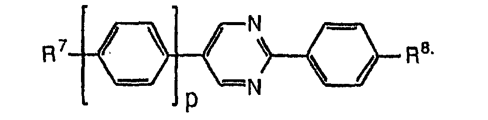 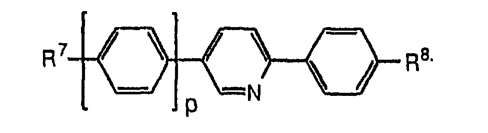 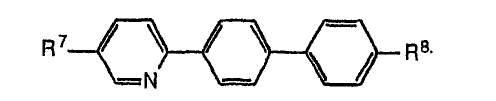 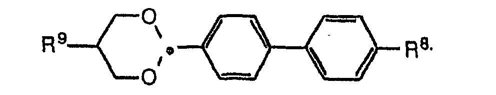 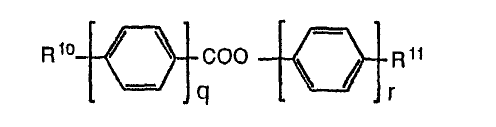 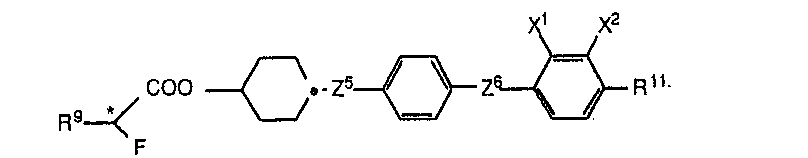 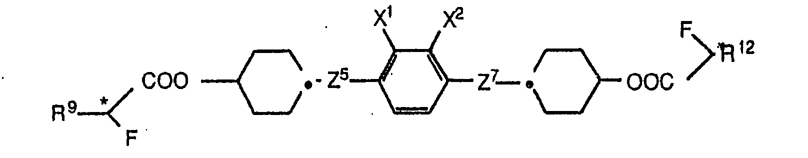 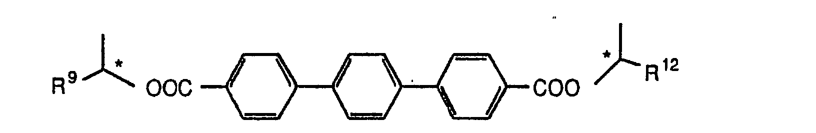 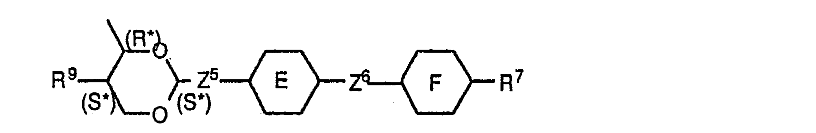 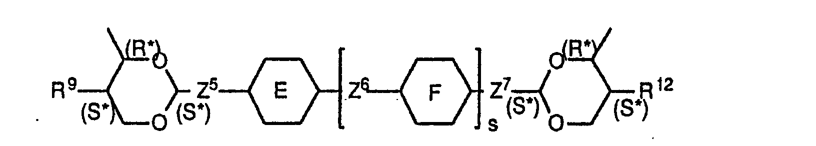 in which
p is 0 or 1;
q and r are 1 or 2, q + r = 2 or 3;
s is 0, 1 or 2;
R⁷ and R⁸, independently of one another, are alkyl, alkenyl, alkoxy, alkenyloxy, alkoxyalkyl, alkoxyalkoxy, alkanoyloxy, alkenoyloxy, alkoxycarbonyl, alkenyloxycarbonyl;
R⁹ and R¹², independently of one another, are alkyl or alkenyl;
R¹⁰ and R¹¹, independently of one another, are alkyl, alkenyl, alkoxy and/or alkenyloxy;
Z⁵, Z⁶ and Z⁷, independently of one another, are a single bond, CH₂-CH₂ or CH₂O and Z⁶ is furthermore OCH₂, COO or OOC;
E and F, independently of one another, are phenylene-1,4-diyl or transcyclohexylene-1,4-diyl;
X¹ and X², independently of one another, are hydrogen or fluorine;
(R*) and (S*) denote the relative configurations.

10. An optical or electro-optical apparatus containing at least one imidazothiazole derivative of the formula I.

11. An optical or electro-optical apparatus containing a liquid crystalline mixture as claimed in any of claims 7, 8 and 9.

## Revendications

1. Dérivés d'imidazothiazole de formule générale 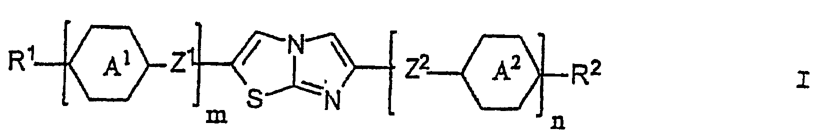 dans laquelle les symboles m, n, R¹ et R² ont la signification suivante :
m et n 0 ou 1, dans laquelle m + n = 1 ou 2,
R¹ R³ ou Q¹ ou, au cas où n=0 et m=1 également R³-A³-Z³-,
R² R⁴ ou Q² ou, au cas où n = 1 et m = 0 également R⁴-A⁴-Z⁴-,
dans laquelle indépendamment les uns des autres
R³ et R⁴ représentent chacun un groupe alkyle ou alcényle ayant chacun 3 à 12 atomes de carbone, dans lequel éventuellement un groupe méthylène ou plusieurs groupes méthylène non voisins sont remplacés par -O-, -COO-, ou -OOC- et/ou un atome d'hydrogène ou plusieurs atomes d'hydrogène par fluor ou chlore,
Q¹ et Q² représentent chacun un des groupes chiraux suivants 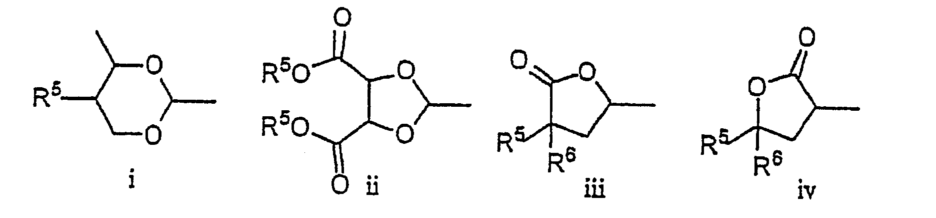 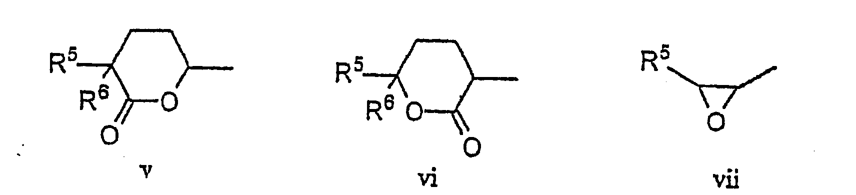 dans lesquels R⁵ et R⁶ représentent chacun un groupe alkyle ou alcényle, dans lequel un groupe méthylène ou plusieurs groupes méthylène non voisins - avec la condition que les atomes d'oxygène ne soient pas reliés directement avec le cycle dans Q¹ ou Q² - peuvent être remplacés par -O-, -COO-, ou -OOC- et/ou un atome d'hydrogène ou plusieurs atomes d'hydrogène par fluor ou chlore,
A¹, A², A³ et A⁴ représentent un trans-1,4-cyclohexylène ou 1,4-phénylène non substitué ou mono- ou di-substitué avec fluor, chlore, cyano ou méthyle, et
Z¹, Z², Z³ et Z⁴ désignent chacun une simple liaison, -CH₂-CH₂-, -O-CH₂-, -CH₂-O-, -COO-, -OOC-, -C≡C-, -(CH₂)₄-, -O-(CH₂)₃-, -CH=CH-CH₂O-,-OCH₂-CH=CH-, -CH=CH-(CH₂)₂-ou (CH₂)₂-CH=CH-.

2. Dérivé d'imidazothiazole selon la revendication 1, **caractérisé en ce qu'**il est optiquement inactif et forme une phase smectique basculée.

3. Dérivé d'imidazothiazole selon la revendication 1, **caractérisé en ce qu'**il est optiquement actif.

4. Dérivé d'imidazothiazole selon une des revendications 1 à 3, **caractérisé en ce que** les groupes R⁵ et R⁶ sont à chaîne linéaire ou ramifiée et possèdent chacun 3 à 12 atomes de carbone.

5. Dérivé d'imidazothiazole selon une des revendications 1 à 4, **caractérisé en ce que** A¹ et/ou A² représente un 1,4-phénylène, 2-fluoro-1,4-phénylène, 3-fluoro-1,4-phénylène ou 2,3-difluoro-1,4-phénylène.

6. Dérivé d'imidazothiazole selon une des revendications 1 à 5, **caractérisé en ce que** Q¹ et/ou Q² répondent à la formule générale i et/ou ii.

7. Mélanges pour cristaux liquides, constitués d'au moins deux composants, **caractérisés par le fait qu'**il au moins un dérivé d'imidazothiazole de formule générale I.

8. Mélange pour cristaux liquides selon la revendication 7, constitué d'au moins une substance dopante optiquement active et d'un matériau pour cristaux liquides avec au moins un composant achiral, **caractérisé en ce que** ladite au moins une substance dopante est formée d'un dérivé chiral d'imidazothiazole de formule générale I et/ou ledit au moins un composant est formé d'un dérivé achiral d'imidazothiazole de formule générale I.

9. Mélange pour cristaux liquides selon la revendication 7 ou 8, **caractérisé en ce qu'**il contient au moins un composé choisi dans le groupe de composés ayant les formules générales 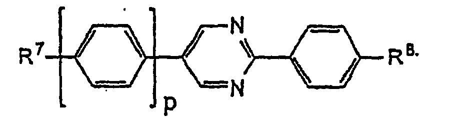 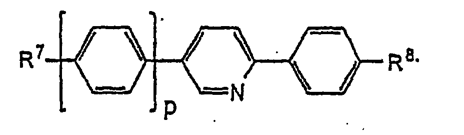 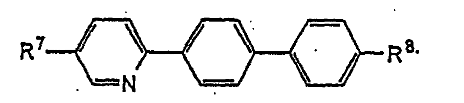 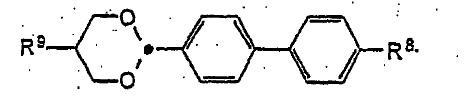 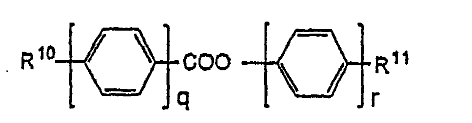 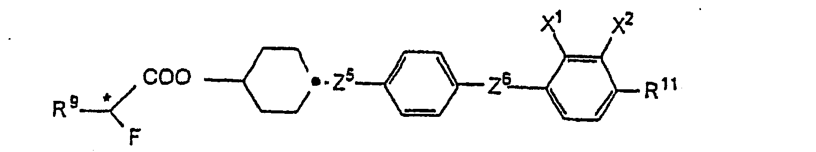 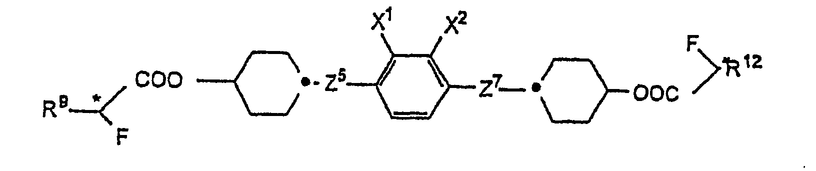 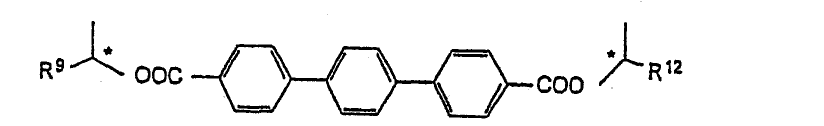 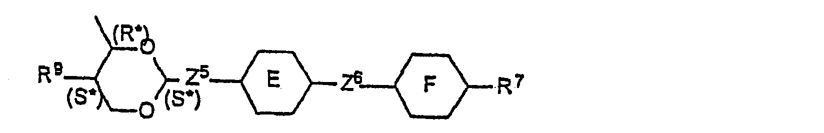 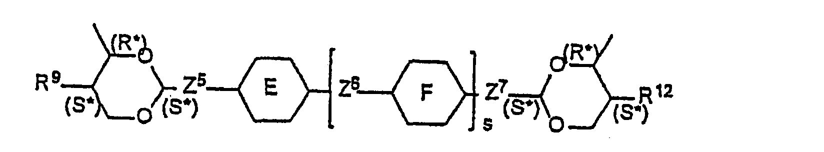 dans lesquelles
p représente 0 ou 1 ;
q et r représentent 1 ou 2, q + r = 2 ou 3 ;
s représente 0, 1 ou 2 ;
R⁷ et R⁸ représentent indépendamment l'un de l'autre un alkyle, alcényle, alcoxy, alcényloxy, alcoxyalkyle, alcoxyalcoxy, alcanoyloxy, alcénoyloxy, alcoxycarbonyle, alcényloxycarbonyle ;
R⁹ et R¹² représentent indépendamment l'un de l'autre un alkyle ou alcényle ;
R¹⁰ et R¹¹ représentent indépendamment l'un de l'autre un allyle, alcényle, alcoxy et/ou alcényloxy ;
Z⁵, Z⁶, Z⁷ représentent indépendamment l'un de l'autre une simple liaison, CH₂-CH₂⁻ ou CH₂O, et Z⁶ également OCH₂, COO ou OOC ;
E et F représentent indépendamment l'un de l'autre un phénylène-1,4-diyle ou trans-cyclohexylène-1,4-diyle ;
X¹ et X² représentent indépendamment l'un de l'autre un hydrogène ou fluor ;
(R*) et (S*) représentent les configurations relatives.

10. Dispositifs optiques ou électrooptiques contenant au moins un dérivé imidazo-thiazole de formule générale I.

11. Dispositifs optiques ou électrooptiques contenant un mélange pour cristaux liquides selon une des revendications 7, 8 ou 9.
